# EUROPÄISCHE PATENTSCHRIFT

(11) **EP 3 102 256 B1**
(45) Veröffentlichungstag und Bekanntmachung des Hinweises auf die Patenterteilung: **27.03.2019**
(21) Anmeldenummer: 15702373.0
(22) Anmeldetag: 03.02.2015
(51) Int. Cl.: A61M 5/31, A61M 5/20, A61M 5/315, A61M 5/24

(54) **INJEKTIONSGERÄT**
INJECTION DEVICE
DISPOSITIF D'INJECTION

(30) Priorität: 05.02.2014 DE 202014001134 U
(43) Veröffentlichungstag der Anmeldung: 14.12.2016
(73) Patentinhaber: Haselmeier AG, 9001 St. Gallen (CH)
(72) Erfinder: KEITEL, Joachim, 73728 Esslingen (DE)
(74) Vertreter: Reinhardt, Annette
(86) Internationale Anmeldenummer: PCT/EP2015/000205
(87) Internationale Veröffentlichungsnummer: WO 2015/117746

(56) Entgegenhaltungen:
- WO-A1-2013/117332
- US-A- 5 114 406
- US-A1- 2009 048 561

## Beschreibung

Die Erfindung betrifft ein Injektionsgerät der im Oberbegriff des Anspruchs 1 angegebenen Gattung.

Aus der WO 2013/117332 A1 ist ein Injektionsgerät bekannt, bei dem ein Rastteil vorgesehen ist, das drehfest mit dem Bedienknopf verbunden ist und mit der Injektionshülse zusammenwirkt. Der Bedienknopf wird beim Einstellen einer auszupressenden Menge an Injektionsflüssigkeit gegenüber dem Gehäuse gedreht und ist beim Auspressen der Injektionsflüssigkeit drehfest gegenüber dem Gehäuse gehalten und in Längsrichtung des Injektionsgeräts geführt. Die Injektionshülse bewegt sich beim Einstellen einer auszupressenden Menge an Injektionsflüssigkeit und beim Auspressen der Injektionsflüssigkeit aus dem Behälter in Richtung der Längsmittelachse des Injektionsgerätes, ohne sich gegenüber dem Gehäuse zu drehen. Dadurch verändert sich die Drehlage des Bedienknopfes gegenüber der Injektionshülse bei jedem Injektionsvorgang.

Eine zweite Rasteinrichtung der WO 2013/117332 A1 wirkt zwischen einem Gehäuseteil und einem Dosierorgan. Das Dosierorgan dreht sich beim Einstellen der auszupressenden Menge einer Injektionsflüssigkeit, und beim Auspressen der auszupressenden Menge an Injektionsflüssigkeit dreht sich das Dosierorgan zurück. Die Rasteinrichtung besitzt zwei einander gegenüberliegend angeordnete Rastarme. Da das Dosierorgan um mehrere Umdrehungen um die Längsmittelachse drehbar ist, wird beim Einstellen der Maximaldosis jede Raststellung mehrfach erreicht.

Werden für eine Therapie beispielsweise einzustellende Mengen an Injektionsflüssigkeit von 0,20 ml und 0,25 ml benötigt, so sind bekannte Injektionsgeräte so ausgelegt, dass Dosierschritte von höchstens 0,05 ml einstellbar sind. Das bedeutet zum einen, dass der Anwender mehrere Rastschritte überwinden muss, bis er die kleinste für die Therapie vorgesehene Dosis erreicht. Zum anderen ist bei einem kleinsten festen Dosierschritt von beispielsweise 0,05 ml die beim Priming-Vorgang zu verwerfende Menge an Injektionsflüssigkeit vergleichsweise groß. Für den Priming-Vorgang wären deshalb deutlich kleinere Dosierschritte wünschenswert. Dies führt allerdings zu einer deutlich erhöhten Anzahl von Raststellungen, die der Bediener beim Einstellen der Dosis überwinden muss.

Der vorliegenden Erfindung liegt die Aufgabe zugrunde, ein Injektionsgerät der gattungsgemäßen Art zu schaffen, das die Anordnung mehrerer Raststellungen in unterschiedlichen Abständen ermöglicht.

Diese Aufgabe wird durch ein Injektionsgerät mit den Merkmalen des Anspruchs 1 gelöst.

Die vorliegende Erfindung sieht vor, dass die Rasteinrichtung zwischen zwei Bauteilen des Injektionsgeräts wirkt, bei denen jeder relativen Lage der beiden Bauteile zueinander eindeutig eine eingestellte Menge an Injektionsflüssigkeit zugeordnet ist. Dadurch können die benötigten Raststellungen mit unterschiedlichen Abständen zueinander angeordnet werden. Beispielsweise könnte für die eingangs beispielhaft beschriebene Therapie ein Injektionsgerät vorgesehen werden, das genau drei Raststellungen bei 0,01 ml für den Priming-Vorgang und 0,20 ml und 0,25 ml für die zu injizierenden Dosen bereitstellt. Dadurch wird die Bedienung des Injektionsgeräts erheblich vereinfacht. Wird im Folgenden auf "die beiden Bauteile" Bezug genommen, sind die beiden Bauteile gemeint, zwischen denen die Rasteinrichtung wirkt und bei denen jeder relativen Lage zueinander eindeutig eine eingestellte Menge an Injektionsflüssigkeit zugeordnet ist.

Vorteilhaft weist die Rasteinrichtung mindestens ein Rastelement und mindestens ein mit dem Rastelement in einer Raststellung zusammenwirkendes Gegenrastelement auf.

Vorteilhaft ist eines der beiden Bauteile, zwischen denen die Rasteinrichtung wirkt, das Dosierorgan und das andere der beiden Bauteile die Injektionshülse. Dosierorgan und Injektionshülse bewegen sich beim Einstellen der auszupressenden Menge an Injektionsflüssigkeit schraubenlinienförmig relativ zueinander, da das Dosierorgan eine Drehbewegung und die Injektionshülse eine Bewegung in Richtung der Längsmittelachse des Injektionsgerätes ausführt. Beim Auspressen der Injektionsflüssigkeit bewegen sich Dosierorgan und Injektionshülse relativ zueinander in ihre Ausgangslage zurück. Dadurch ist jeder relativen Lage von Dosierorgan und Injektionshülse zueinander genau eine eingestellte Menge an Injektionsflüssigkeit zugeordnet.

Es kann auch vorgesehen sein, dass eines der beiden Bauteile, zwischen denen die Rasteinrichtung wirkt, die Injektionshülse und das andere der beiden Bauteile das Gehäuse ist. Die Injektionshülse bewegt sich beim Einstellen einer auszupressenden Menge an Injektionsflüssigkeit in Richtung der Längsmittelachse des Injektionsgerätes. Beim Auspressen der Injektionsflüssigkeit bewegt sich die Injektionshülse zurück in ihre Ausgangslage. Dadurch entspricht jeder axialen Lage der Injektionshülse genau eine eingestellte Menge an Injektionsflüssigkeit.

Die Angabe, dass die Rasteinrichtung zwischen den beiden Bauteilen wirkt, bedeutet, dass die Rasteinrichtung zwischen diesen Bauteilen wirksam ist, aber nicht, dass Rastelement und Gegenrastelement an den beiden Bauteilen selbst angeordnet sein müssen. Rastelement und Gegenrastelement können vielmehr an weiteren Bauteilen ausgebildet sein, die die Relativbewegung der beiden Bauteile zueinander ebenfalls ausführen und dadurch zwischen den beiden Bauteilen wirksam sind.

Es ist vorgesehen, dass das Injektionsgerät ein Bedienelement besitzt. Das Bedienelement besitzt vorteilhaft eine distale Stellung, in der sich das Bedienelement beim Einstellen der auszupressenden Menge an Injektionsflüssigkeit befindet, sowie eine proximale Stellung, in der sich das Bedienelement beim Auspressen der auszupressenden Menge an Injektionsflüssigkeit befindet. Die distale Stellung und die proximale Stellung des Bedienelements sind dabei Positionen des Bedienelements relativ zur Injektionshülse. Das Bedienelement ist vorteilhaft über eine erste Kupplung drehfest mit einem drehfest mit dem Dosierorgan verbundenen Mitnehmer und über eine zweite Kupplung mit der Injektionshülse verbindbar. In der distalen Stellung des Bedienelements ist das Bedienelement vorteilhaft über die erste Kupplung drehfest mit dem Mitnehmer verbunden. Die zweite Kupplung ist vorteilhaft in der distalen Stellung des Bedienelements geöffnet, so dass das Bedienelement gegenüber der Injektionshülse drehbar ist. In der proximalen Stellung des Bedienelementes ist vorteilhaft die erste Kupplung geöffnet, und das Bedienelement ist gegenüber dem Mitnehmer drehbar, und das Bedienelement ist über die zweite Kupplung drehfest mit der Injektionshülse verbunden. Beim Einstellen einer einzustellenden Menge an Injektionsflüssigkeit wird das Bedienelement gedreht. Mit dem Bedienelement drehen sich der Mitnehmer, das Dosierorgan und der drehfest mit dem Bedienelement verbundene Dosierkolben. Beim Auspressen von Injektionsflüssigkeit ist das Bedienelement drehfest mit der Injektionshülse verbunden und dadurch drehfest gegenüber dem Gehäuse geführt. Der Mitnehmer dreht sich gemeinsam mit dem Dosierorgan um die Längsmittelachse des Injektionsgerätes und bewegt über die zweite Gewindeverbindung den Dosierkolben in proximale Richtung. Dadurch wird die Injektionsflüssigkeit aus dem Behälter ausgepresst.

Eine angenehme Bedienung ergibt sich, wenn die Rasteinrichtung nur beim Einstellen der auszupressenden Menge an Injektionsflüssigkeit, nicht aber beim Injektionsvorgang wirksam ist. Dies kann auf einfache Weise dadurch erreicht werden, dass die Rasteinrichtung an die Stellung des Bedienelements gekoppelt ist und in der distalen Stellung des Bedienelements wirksam ist. In der proximalen Stellung des Bedienelements sind das mindestens eine Rastelement und das mindestens eine Gegenrastelement vorteilhaft unabhängig von der relativen Lage der beiden Bauteile zueinander außer Eingriff. Wenn die Rasteinrichtung nur beim Einstellen einer auszupressenden Menge an Injektionsflüssigkeit wirksam ist, kann das Rastelemente und/oder das Gegenrastelement unsymmetrisch ausgebildet sein, so dass für das Überwinden einer Raststellung zum Verringern der eingestellten auszupressenden Menge an Injektionsflüssigkeit eine deutlich größere Kraft benötigt wird als zum Einstellen einer auszupressenden Menge an Injektionsflüssigkeit.

Vorteilhaft ist eines der beiden Bauteile, zwischen denen die Rasteinrichtung wirkt, der Mitnehmer und das andere der beiden Bauteile ist die Injektionshülse. Dadurch ergibt sich ein einfacher Aufbau des Injektionsgeräts. Bei Injektionsgeräten, bei denen das Bedienelement bis zum Erreichen der Maximaldosis um weniger als eine Umdrehung gedreht wird, ist jeder relativen Drehlage von Mitnehmer und Injektionshülse genau eine eingestellte Menge an Injektionsflüssigkeit zugeordnet. Der Mitnehmer ist vorteilhaft in Richtung der Längsmittelachse an die Stellung der Injektionshülse gekoppelt, so dass sich der Mitnehmer beim Einstellen der auszupressenden Menge an Injektionsflüssigkeit mit der Injektionshülse in distaler Richtung und beim Auspressen der eingestellten Menge an Injektionsflüssigkeit in proximaler Richtung bewegt. Dadurch wird ein einfacher Aufbau der ersten Kupplung, die zwischen Mitnehmer und Bedienelement wirkt, ermöglicht.

Ein einfacher Aufbau des Injektionsgeräts ergibt sich, wenn mindestens ein Rastelement an dem einen der beiden Bauteile und mindestens ein gegen Gegenrastelement an dem anderen der beiden Bauteile angeordnet ist. Besonders vorteilhaft sind das Rastelement bzw. das Gegenrastelement einteilig mit dem jeweiligen Bauteil ausgebildet. Auch eine feste Verbindung mit dem jeweiligen Bauteil kann vorteilhaft sein, insbesondere zur Vereinfachung der Fertigung des Bauteils.

Um auf einfache Weise zu erreichen, dass die Rasteinrichtung nur beim Einstellen einer einzustellenden Menge an Injektionsflüssigkeit, nicht aber beim Auspressen der Injektionsflüssigkeit wirksam ist, ist vorteilhaft mindestens ein Rastelement an einem Rastteil angeordnet, das mit dem einen der beiden Bauteile drehfest und in Richtung der Längsmittelachse gegenüber diesem Bauteil verschiebbar verbunden ist. Vorteilhaft ist mindestens ein Gegenrastelement an dem anderen der beiden Bauteile angeordnet. Das mindestens eine Rastelement kann in einer ersten axialen Stellung des Rastteils vorteilhaft in Eingriff mit dem mindestens einen Gegenrastelement kommen. In einer zweiten axialen Stellung des Rastteils ist das mindestens eine Rastelement vorteilhaft unabhängig von der relativen Lage der beiden Bauteile zueinander außer Eingriff mit dem mindestens einen Gegenrastelement.

Ein einfacher Aufbau ergibt sich, wenn die Stellung des Rastteils an die Stellung des Bedienelements gekoppelt ist, so dass sich das Rastteil in der distalen Stellung des Bedienelements in seiner ersten axialen Stellung und in der proximalen Stellung des Bedienelements in seiner zweiten axialen Stellung befindet. Vorteilhaft ist das Rastteil in Richtung auf seine erste axiale Stellung elastisch vorgespannt, vorzugsweise durch mindestens eine Feder.

Ein einfacher Aufbau mit einer geringen Anzahl an Einzelteilen ergibt sich, wenn das Rastteil mindestens einen Federarm aufweist, der das Rastteil in Richtung auf seine erste axiale Stellung vorspannt. Der Federarm ist vorteilhaft einteilig mit dem Rastteil ausgebildet, so dass keine zusätzliche Feder zur Vorspannung des Rastteils in Richtung auf seine erste axiale Stellung benötigt wird.

Vorteilhaft wirkt zwischen der Injektionshülse und dem Dosierorgan eine Feder, die das Dosierorgan in der zweiten Drehrichtung vorspannt. Dadurch wird das Dosierorgan in Richtung auf die nächste Raststellung, die der nächstniedrigen einzustellenden Menge an Injektionsflüssigkeit entspricht, zurückgestellt, wenn das Bedienelement zwischen zwei Raststellungen losgelassen wird. Dadurch wird ein versehentliches Auspressen einer nicht vorgesehenen Menge an Injektionsflüssigkeit auf einfache Weise verhindert. Da sich die Injektionshülse gegenüber dem Dosierorgan beim Einstellen einer auszupressenden Menge an Injektionsflüssigkeit in Richtung der Längsmittelachse bewegt, ist die Feder vorteilhaft mit dem drehfest mit dem Dosierorgan verbundenen Mitnehmer verbunden. Vorteilhaft ist die Feder mit einem Ende an der Injektionshülse und mit dem anderen Ende am Mitnehmer festgelegt. Für ein Injektionsgerät, das ein Rastteil besitzt, ergibt sich ein einfacher Aufbau, wenn die Feder mit einem Ende am Rastteil und mit dem anderen Ende am Mitnehmer festgelegt ist. Eine Feder, die das Dosierorgan in der zweiten Drehrichtung vorspannt, ist insbesondere bei einem Injektionsgerät vorteilhaft, dass dessen Rasteinrichtung nur beim Einstellen, nicht aber beim Auspressen einer Menge an Injektionsflüssigkeit wirksam ist. Vorteilhaft ist die Rasteinrichtung so ausgebildet, dass zum Überwinden der Raststellungen beim Einstellen einer auszupressenden Menge an Injektionsflüssigkeit eine kleinere Kraft benötigt wird als zum Zurückdrehen in Gegenrichtung. Dadurch kann sichergestellt werden, dass das Dosierorgan beim Loslassen des Bedienelements zwischen zwei Raststellungen nur bis zur nächstniedrigen Raststellung zurückgestellt wird und diese Raststellung nicht überwinden kann. Gleichzeitig kann die Feder, die das Dosierorgan in der zweiten Drehrichtung vorspannt, so stark ausgelegt werden, dass ein sicheres Zurückdrehen des Dosierorgans auch bei ungünstigen Reibungsverhältnissen und Toleranzen sichergestellt ist.

Ausführungsbeispiele der Erfindung werden im Folgenden anhand der Zeichnung erläutert. Es zeigen:
- Fig. 1: eine Seitenansicht eines ersten Ausführungsbeispiels eines Injektionsgeräts in Nullstellung,
- Fig. 2: einen Schnitt entlang der Linie II-II in Fig. 1,
- Fig. 3: eine Seitenansicht des Injektionsgeräts aus Fig. 1 nach dem Einstellen einer auszupressenden Menge an Injektionsflüssigkeit,
- Fig. 4: einen Schnitt entlang der Linie IV-IV in Fig. 3,
- Fig. 5: eine Seitenansicht des Injektionsgeräts aus Fig. 1 nach dem Einstellen einer auszupressenden Menge an Injektionsflüssigkeit und dem Verschieben des Bedienelements in proximale Richtung,
- Fig. 6: einen Schnitt entlang der Linie VI-VI in Fig. 5,
- Fig. 7: eine Seitenansicht auf den Mitnehmer, die Feder und das Rastteil des Injektionsgeräts aus den Figuren 1 bis 6,
- Fig. 8: eine Seitenansicht auf die Anordnung in Fig. 7 in Richtung des Pfeils VIII in Fig. 7,
- Fig. 9: eine Draufsicht in Richtung des Pfeils IX in Fig. 7,
- Fig. 10: den Ausschnitt X aus Fig. 9 in vergrößerter Darstellung,
- Fig. 11 und 12: perspektivische Darstellungen des Mitnehmers des Injektionsgeräts aus den Figuren 1 bis 6,
- Fig. 13: eine Seitenansicht des Mitnehmers,
- Fig. 14: eine Seitenansicht in Richtung des Pfeils XIV in Fig. 13,
- Fig. 15: einen Schnitt entlang der Linie XV-XV in Fig. 14,
- Fig. 16 und 17: perspektivische Darstellungen des Rastteils des Injektionsgeräts aus den Figuren 1 bis 6,
- Fig. 18: eine Seitenansicht des Rastteils aus den Figuren 16 und 17,
- Fig. 19: einen Schnitt entlang der Linie XIX-XIX in Fig. 18,
- Fig. 20 und 21: perspektivische Darstellungen des Bedienelements des Injektionsgeräts,
- Fig. 22: eine Seitenansicht des Bedienelements,
- Fig. 23: einen Schnitt entlang der Linie XXIII-XXIII in Fig. 22,
- Fig. 24: eine Ansicht auf das Bedienelement in Richtung des Pfeils XXIV in Fig. 22,
- Fig. 25 bis 27: perspektivische Darstellungen der Injektionshülse des Injektionsgeräts,
- Fig. 28 und 29: Seitenansichten der Injektionshülse,
- Fig. 30: einen Schnitt entlang der Linie XXX-XXX in Fig. 29,
- Fig. 31: den Ausschnitt XXXI aus Fig. 30 in vergrößerter Darstellung,
- Fig. 32: einen Schnitt entlang der Linie XXXII-XXXII in Fig. 30,
- Fig. 33 und 34: Seitenansichten des Dosierorgans,
- Fig. 35: einen Schnitt entlang der Linie XXXV-XXXV in Fig. 33,
- Fig. 36: einen Schnitt entlang der Linie XXXVI-XXXVI in Fig. 33,
- Fig. 37: eine perspektivische Darstellung einer Kolbenstange des Injektionsgeräts aus den Figuren 1 bis 6,
- Fig. 38: eine Seitenansicht der Kolbenstange aus Fig. 37,
- Fig. 39: einen Schnitt entlang der Linie XXXIX-XXXIX in Fig. 38,
- Fig. 40 und 41: Seitenansichten eines Gehäuseteils des Injektionsgeräts aus den Figuren 1 bis 6,
- Fig. 42: einen Schnitt entlang der Linie XLII-XLII in Fig. 41,
- Fig. 43: einen Schnitt entlang der Linie XLIII-XLIII in Fig. 41,
- Fig. 44: eine Seitenansicht eines Halters des Injektionsgeräts aus den Figuren 1 bis 6,
- Fig. 45: einen Schnitt entlang der Linie XLV-XLV in Fig. 44,
- Fig. 46: Injektionshülse, Rastteil und Mitnehmer eines Ausführungsbeispiels eines Injektionsgeräts,
- Fig. 47: einen Schnitt entlang der Linie XLVII-XLVII in Fig. 46,
- Fig. 48 bis 52: perspektivische Darstellungen des Rastteils aus den Figuren 46 und 47,
- Fig. 53: eine Seitenansicht eines Ausführungsbeispiels einer Injektionshülse eines Injektionsgeräts,
- Fig. 54: einen Schnitt entlang der Linie LIV-LIV in Fig. 53,
- Fig. 55: einen Schnitt entlang der Linie LV-LV in Fig. 54,
- Fig. 56: eine Seitenansicht eines Ausführungsbeispiels eines Mitnehmers eines Injektionsgeräts,
- Fig. 57: einen Schnitt entlang der Linie LVII-LVII in Fig. 56,
- Fig. 58: den Ausschnitt LVIII in Fig. 57 in vergrößerter Darstellung,
- Fig. 59 und 60: Seitenansichten eines Ausführungsbeispiels eines Injektionsgeräts nach dem Einstellen einer auszupressenden Menge an Injektionsflüssigkeit,
- Fig. 61: einen Schnitt entlang der Linie LXI-LXI in Fig. 60,
- Fig. 62: den Ausschnitt LXII in Fig. 61 in vergrößerter Darstellung,
- Fig. 63: eine perspektivische Darstellung eines Ausführungsbeispiels eines Dosierorgans,
- Fig. 64: den Ausschnitt LXIV aus Fig. 63 in vergrößerter Darstellung,
- Fig. 65: eine Seitenansicht des Dosierorgans aus Fig. 63,
- Fig. 66: einen Schnitt entlang der Linie LXVI-LXVI in Fig. 65,
- Fig. 67: eine Seitenansicht eines Ausführungsbeispiels einer Injektionshülse,
- Fig. 68: einen Schnitt entlang der Linie LXVIII-LXVIII in Fig. 67,
- Fig. 69: einen Schnitt entlang der Linie LXIX-LXIX in Fig. 67,
- Fig. 70: eine Seitenansicht eines Injektionsgeräts in Nullstellung,
- Fig. 71: einen Schnitt entlang der Linie LXXI-LXXI in Fig. 70,
- Fig. 72: eine Seitenansicht des Injektionsgeräts aus Fig. 70 nach dem Einstellen einer auszupressenden Menge an Injektionsflüssigkeit,
- Fig. 73: einen Schnitt entlang der Linie LXXIII-LXXIII in Fig. 72.

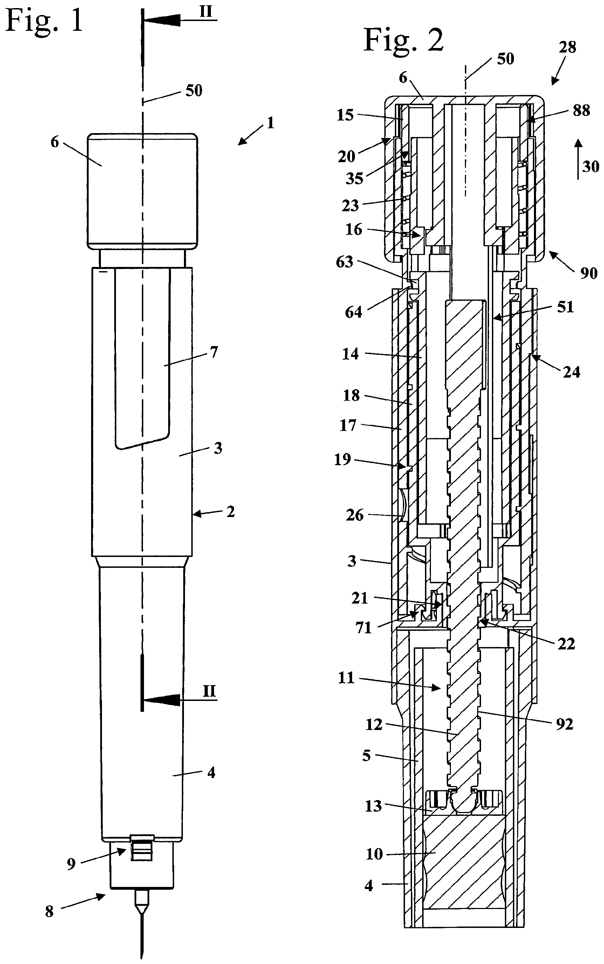

Fig. 1 zeigt ein Injektionsgerät 1, das ein Gehäuse 2 besitzt. Das Gehäuse 2 umfasst ein oberes, distales Gehäuseteil 3 sowie einen an der proximalen Seite des oberen Gehäuseteils 3 angeordneten Halter 4. An der proximalen Seite des Halters 4 ist eine Injektionsnadel 8 festgelegt. Benachbart zur Injektionsnadel 8 weist der Halter 4 eine Rasteinrichtung 9 auf, an der ein in Fig. 2 gezeigter Behälter 5 im Halter 4 verrastet ist. An der distalen Seite des Injektionsgeräts 1 ist ein Bedienelement 6 angeordnet. Wie Fig. 1 zeigt, besitzt das Injektionsgerät 1 eine Längsmittelachse 50, die in Längsrichtung des Injektionsgeräts 1 verläuft. Das obere Gehäuseteil 3 besitzt ein Sichtfenster 7, das mindestens teilweise durchsichtig ausgebildet ist. In Fig. 1 ist das Sichtfenster 7 schematisch und nicht durchsichtig gezeichnet, so dass die darunter liegenden Komponenten in Fig. 1 nicht sichtbar sind.

Das distale Ende des Injektionsgeräts 1 ist das einer am Injektionsgerät gehaltenen Injektionsnadel 8 abgewandt liegende Ende. Mit "proximal" wird die Seite des Injektionsgeräts 1 bezeichnet, die bei einer Injektion der Einstichstelle zugewandt liegt, und mit "distal" die Seite, die der Einstichstelle abgewandt liegt. Die proximale Richtung bezeichnet die Injektionsrichtung, also die Richtung zur Injektionsnadel 8 hin bzw. die Richtung, in der die Injektionsflüssigkeit aus dem Behälter 5 ausgepresst wird. Die distale Richtung bezeichnet die entgegengesetzte Richtung, also von der Injektionsnadel 8 weg.

Wie Fig. 2 zeigt, ist im Behälter 5 ein Stopfen 10 angeordnet, an dem eine Kolbenscheibe 13 eines Dosierkolbens 11 anliegt. Der Dosierkolben 11 umfasst außerdem eine Kolbenstange 12, die ein Außengewinde 92 trägt.

Im oberen Gehäuseteil 3 ist eine Injektionshülse 17 angeordnet, deren Außenseite durch das Sichtfenster 7 des oberen Gehäuseteils 3 sichtbar ist. Die Injektionshülse 17 besitzt eine Öffnung 26, durch die der Außenumfang eines innerhalb der Injektionshülse 17 angeordneten Dosierorgans 18 sichtbar ist. Das Dosierorgan 18, das auch als Skalenrohr bezeichnet werden kann, trägt an seinem Außenumfang eine in Fig. 2 nicht sichtbare Skala, die durch das Sichtfenster 7 und die Öffnung 26 für den Bediener sichtbar ist.

Die Injektionshülse 17 ist im oberen Gehäuseteil 3 in Richtung der Längsmittelachse 50 verschiebbar und gegenüber dem oberen Gehäuseteil 3 drehfest gehalten. Das Dosierorgan 18 und die Injektionshülse 17 sind über eine erste Gewindeverbindung 19 miteinander verbunden. Innerhalb des Dosierorgans 18 ist ein Mitnehmer 14 angeordnet, der drehfest mit dem Dosierorgan 18 verbunden ist. Der Mitnehmer 14 besitzt eine Umfangsnut 63, in die ein Halterand 64 der Injektionshülse 17 ragt. Der Halterand 64 ist dabei mit Spiel in der Umfangsnut 63 gehalten. Dadurch sind die Injektionshülse 17 und der Mitnehmer 14 in Richtung der Längsmittelachse 50 aneinander gekoppelt. Geringfügige Relativbewegungen zwischen Injektionshülse 17 und Mitnehmer 14 in Richtung der Längsmittelachse 50 sind aufgrund des Spiels jedoch möglich.

Das Dosierorgan 18 ist über eine Verrastung 71 in Richtung der Längsmittelachse 50 fest im oberen Gehäuseteil 3 gehalten. Im Ausführungsbeispiel ist die Verrastung 71 am proximalen Ende des Dosierorgans 18 angeordnet. Das Dosierorgan 18 ist über ein Drehlager 21 drehbar im oberen Gehäuseteil 3 gelagert. Das Dosierorgan 18 ist über eine zweite Gewindeverbindung 22 mit der Kolbenstange 12 des Dosierkolbens 11 verbunden. Das Bedienelement 6 ist über einen Mitnahmeabschnitt 51 drehfest mit der Kolbenstange 12 verbunden.

Das Bedienelement 6 ist in Fig. 2 in seiner distalen Stellung 90 relativ zur Injektionshülse 17 gezeigt. Das Bedienelement 6 ist über eine erste Kupplung 16 mit dem Mitnehmer 14 verbindbar. In der distalen Stellung 90 des Bedienelements 6 sind Bedienelement 6 und Mitnehmer 14 über die erste Kupplung 16 drehfest miteinander verbunden. Im Bedienelement 6 ist ein Rastteil 15 angeordnet. Im Ausführungsbeispiel ist das Rastteil 15 ringförmig ausgebildet und am Außenumfang des Mitnehmers 14 angeordnet. Zwischen dem Mitnehmer 14 und dem Rastteil 15 ist eine Rasteinrichtung 35 gebildet, die im Folgenden noch näher beschrieben wird. Das Rastteil 15 ist drehfest mit der Injektionshülse 17 verbunden. Das Rastteil 15 ist in Richtung der Längsmittelachse 50 beweglich und von einer Feder 23 in Richtung auf seine in Fig. 2 gezeigte erste axiale Stellung 88 vorgespannt.

Zwischen dem Bedienelement 6 und der Injektionshülse 17 ist eine zweite Kupplung 20 vorgesehen, die in der in Fig. 2 gezeigten Nullstellung 28 des Injektionsgeräts 1 geöffnet ist. Dadurch ist das Bedienelement 6 gegenüber der Injektionshülse 17 drehbar. In der Nullstellung 28 ist keine Dosis eingestellt. In der Nullstellung 28 liegt die Injektionshülse 17 an einem ersten Anschlag 24 am oberen Gehäuseteil 3 an.

Zum Einstellen einer auszupressenden Menge an Injektionsflüssigkeit dreht der Bediener das Bedienelement 6 in einer ersten Drehrichtung, im Ausführungsbeispiel im Uhrzeigersinn. Über die erste Kupplung 16 drehen sich der Mitnehmer 14 und das drehfest mit dem Mitnehmer 14 verbundene Dosierorgan 18 mit. Über den Mitnahmeabschnitt 51 des Bedienelements 6 wird auch die Kolbenstange 12 mitgedreht. Aufgrund der ersten Gewindeverbindung 19 und der drehfesten Fixierung der Injektionshülse 17 im oberen Gehäuseteil 3 wird die Injektionshülse 17 in distale Richtung 30 bewegt. Mit der Injektionshülse 17 bewegen sich auch der Mitnehmer 14 sowie das Bedienelement 6 in distale Richtung. Da sich der Mitnehmer 14 gegenüber dem drehfest mit der Injektionshülse 17 verbundenen Rastteil 15 dreht, sind Rastschritte der Rasteinrichtung 35 für den Bediener spür- und hörbar. Die Rasteinrichtung 35 ist beim Einstellen einer auszupressenden Menge an Injektionsflüssigkeit wirksam.

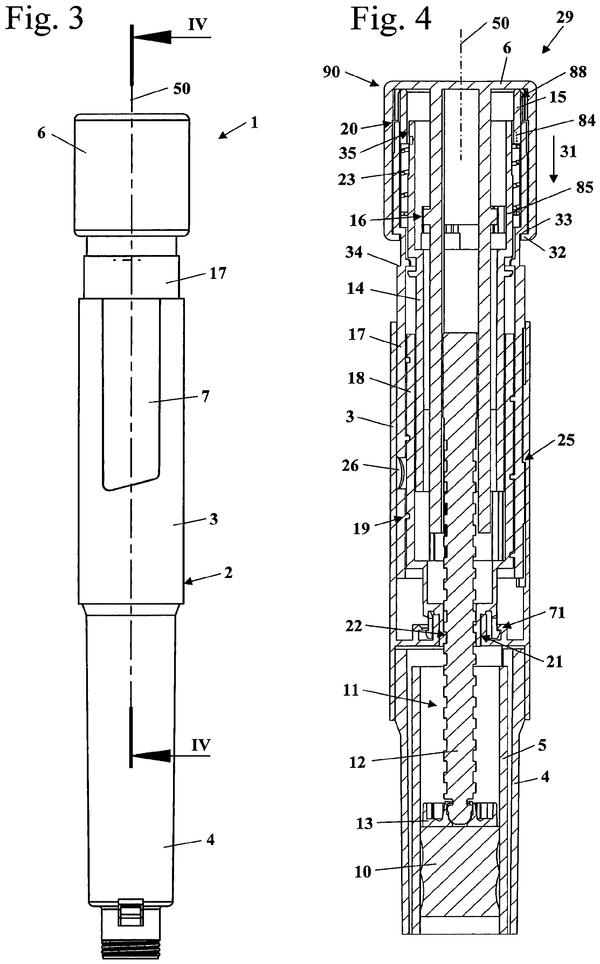

Die Figuren 3 und 4 zeigen das Injektionsgerät 1 in Injektionsstellung 29 nach dem Einstellen einer auszupressenden Menge an Injektionsflüssigkeit. Das Bedienelement 6 wurde dabei um weniger als eine volle Umdrehung gegenüber dem oberen Gehäuseteil 3 gedreht. Die Injektionshülse 17 hat sich in distaler Richtung teilweise aus dem oberen Gehäuseteil 3 herausbewegt. Wie Fig. 4 zeigt, ist die Feder 23 nicht nur als Druckfeder ausgebildet, sondern wirkt zusätzlich als Torsionsfeder. Hierzu ist die Feder 23 mit einem ersten Ende 84 am Rastteil 15 und mit einem zweiten Ende 85 am Mitnehmer 14 eingehängt. Beim Drehen des Bedienelements 6 in der ersten Drehrichtung wird die Feder 23 gespannt. Wird das Bedienelement 6 vom Bediener zwischen zwei Raststellungen der Rasteinrichtung 35 losgelassen, so dreht die Feder 23 den Mitnehmer 14 und mit dem Mitnehmer das Dosierorgan 18 in die nächstniedrige Raststellung, also die Raststellung, die der nächstniedrigen vorgesehenen Menge an Injektionsflüssigkeit entspricht, zurück. Die Feder 23 spannt außerdem das Rastteil 15 in seine erste axiale Stellung 88 vor. Am Rastteil 15 liegt das Bedienelement 6 an, das über das Rastteil 15 ebenfalls in seine distale Stellung 90 vorgespannt ist.

Zum Auspressen der eingestellten Menge an Injektionsflüssigkeit muss der Bediener das Bedienelement 6 entgegen der in Richtung der Längsmittelachse 50 wirkenden Kraft der Feder 23 in proximale Richtung 31 bewegen. Wie Fig. 4 zeigt, besitzt das Bedienelement 6 ein Anschlagelement 32, das in der in Fig. 4 gezeigten Stellung an einem ersten Anschlag 33 der Injektionshülse 17 anliegt. Das Anschlagelement 32 liegt dabei an der proximalen Seite des ersten Anschlags 33 und wird von der Feder 23 gegen den ersten Anschlag 33 gedrückt. Wie Fig. 4 auch zeigt, besitzt die Injektionshülse 17 einen zweiten Anschlag 34, der an der proximalen Seite des Anschlagelements 32 und in der in Fig. 4 gezeigten Stellung in einem Abstand zum Anschlagelement 32 angeordnet ist. Wie Fig. 4 auch zeigt, liegt die Injektionshülse 17 in der in Fig. 4 gezeigten Injektionsstellung 29 an einem Anschlag 25 des oberen Gehäuseteils 3 an, der die maximal einzustellende Menge an Injektionsflüssigkeit begrenzt.

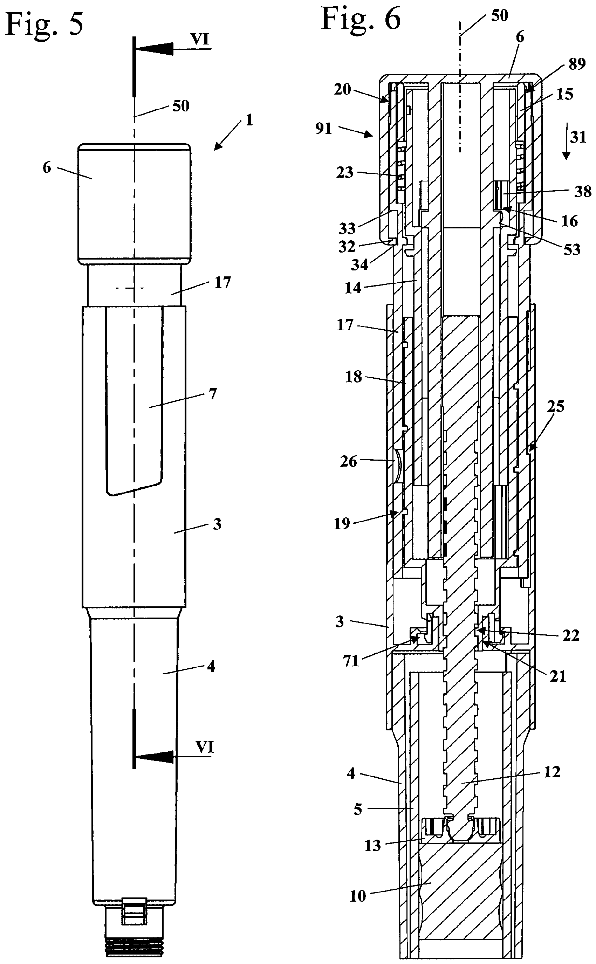

Die Figuren 5 und 6 zeigen das Injektionsgerät 1 nach dem Verschieben des Bedienelements 6 aus seiner distalen Stellung 90 in seine proximale Stellung 91 und vor dem Auspressen von Injektionsflüssigkeit. In der proximalen Stellung 91 des Bedienelements 6 liegt das Anschlagelement 32 am zweiten Anschlag 34 an. Wie Fig. 6 auch zeigt, wurde das Rastteil 15 vom Bedienelement 6 in proximale Richtung 31 in seine zweite axiale Stellung 89 verschoben. In dieser Stellung ist die Rasteinrichtung 35 nicht aktiv, so dass beim Auspressen von Injektionsflüssigkeit keine Raststellungen zu hören oder zu fühlen sind.

Die erste Kupplung 16 ist in der proximalen Stellung 91 des Bedienelements geöffnet. Dadurch kann sich der Mitnehmer 14 gegenüber dem Bedienelement 6 drehen. Wie Fig. 6 auch zeigt, besitzt die erste Kupplung 16 Rastzähne 38 am Mitnehmer 14, die bei geschlossener Kupplung 16 zwischen die Rastzähne 53 des Bedienelements 6 eingreifen. Bei geöffneter Kupplung 16 sind die Rastzähne 38 in Richtung der Längsmittelachse 50 beabstandet von den Rastzähnen 53 angeordnet und außer Eingriff miteinander. Die zweite Kupplung 20 ist in der proximalen Stellung 91 des Bedienelements 6 geschlossen, so dass das Bedienelement 6 drehfest mit der Injektionshülse 17 und damit auch drehfest mit dem oberen Gehäuseteil 3 verbunden ist. Wird das Bedienelement 6 aus der in Fig. 6 gezeigten Stellung in proximaler Richtung 31 verschoben, so bewegt sich die Injektionshülse 17 über das Anschlagelement 32 und den zweiten Anschlag 34 in proximale Richtung. Über die erste Gewindeverbindung 19 wird das Dosierorgan 18 gedreht. Die Kolbenstange 12 ist drehfest mit dem Bedienelement 6 und über das Bedienelement 6 drehfest mit dem oberen Gehäuseteil 3 verbunden. Aufgrund der Drehung des Dosierorgans 18 wird die Kolbenstange 12 in proximale Richtung bewegt und presst dadurch die eingestellte Menge an Injektionsflüssigkeit aus dem Behälter 5 aus. Der Mitnehmer 14 wird von der Injektionshülse 17 in proximale Richtung mitgenommen. Beim Bewegen des Bedienelements 6 in proximale Richtung 31 entspannt sich die Feder 23 mindestens teilweise und unterstützt dadurch den Injektionsvorgang.

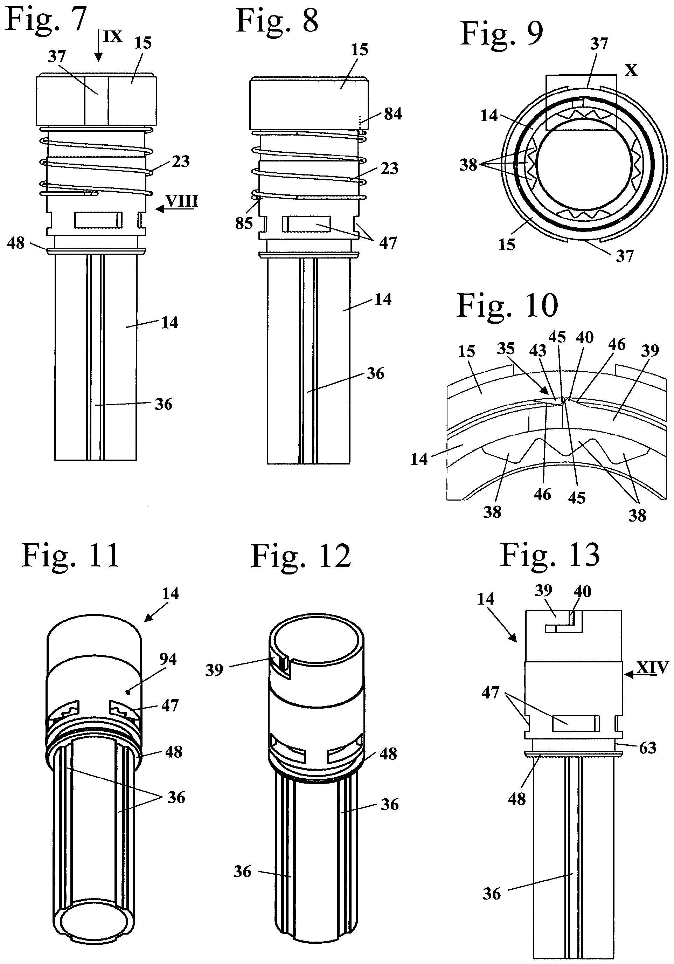

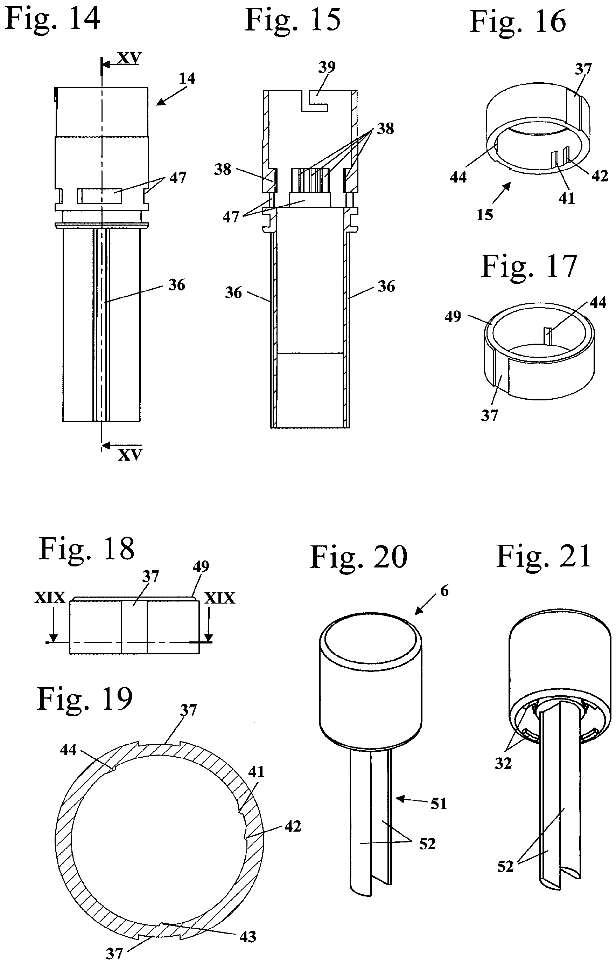

Die Figuren 7 bis 10 zeigen den Mitnehmer 14, das Rastteil 15 und die Feder 23. Fig. 8 zeigt schematisch die Einhängung der beiden Enden 84 und 85 der Feder 23 am Rastteil 15 und am Mitnehmer 14. Das Rastteil 15 besitzt Längsnuten 37 zur drehfesten Verbindung mit der Injektionshülse 17. Der Mitnehmer 14 besitzt an seinem proximalen, zylindrischen Abschnitt Längsnuten 36 zur drehfesten Verbindung mit dem Dosierorgan 18. Dieser zylindrische, proximale Bereich wird an seiner distalen Seite von einem Rand 48 begrenzt. Der Mitnehmer 14 besitzt, wie die Figuren 9 und 10 zeigen, die nach innen ragenden Rastzähne 38. Die Rastzähne 38 erstrecken sich nicht über den gesamten Innenumfang des Mitnehmers 14. Im Ausführungsbeispiel sind vier Gruppen von jeweils drei Rastzähnen 38 vorgesehen, die symmetrisch angeordnet sind. Auch eine andere Anzahl oder Anordnung der Rastzähne 38 kann vorteilhaft sein. Jeweils an der proximalen Seite der Rastzähne 38 weist der Mitnehmer 14 eine Öffnung 47 auf, wie auch die Figuren 14 und 15 zeigen. Dadurch kann das proximale Ende der Rastzähne 38 fertigungstechnisch einfach hergestellt werden. Im Bereich der Öffnungen 47 befinden sich die in Fig. 6 gezeigten Rastzähne 53 des Bedienelements 6 bei geöffneter erster Kupplung 16. Die Feder 23 ist als kombinierte Druck- und Torsionsfeder ausgebildet und spannt das Rastteil 15 in distale Richtung und den Mitnehmer 14 in Drehrichtung um die Längsmittelachse 50 in Richtung auf die Nullstellung 28 vor.

Die Figuren 9 und 10 zeigen auch die Rasteinrichtung 35 im Einzelnen. Die Rasteinrichtung 35 umfasst ein am Rastteil 15 ausgebildetes Rastelement 43. Mit dem Rastelement 43 wirkt ein Gegenrastelement 40 am Mitnehmer 14 zusammen. Das Gegenrastelement 40 ist an einem Rastarm 39 ausgebildet und aufgrund der Eigenelastizität des Materials federnd. Sowohl das Rastelement 43 als auch das Gegenrastelement 40 sind in Umfangsrichtung unsymmetrisch ausgebildet. Das Gegenrastelement 40 besitzt eine Rastflanke 45, hinter der das Rastelement 43 in Raststellung zu liegen kommt. Die weitere Flanke des Gegenrastelements 40 ist als Führungsflanke 46 ausgebildet, die vergleichsweise flach verläuft. Entsprechend ist auch das Gegenrastelement 43 unsymmetrisch ausgebildet und besitzt eine Rastflanke 45 sowie eine Führungsflanke 46. Beim Einstellen einer auszupressenden Menge an Injektionsflüssigkeit wird der Mitnehmer 14 zusammen mit dem Bedienelement 6 in einer ersten Drehrichtung gedreht. Bei dieser Drehung kommen die Führungsflanken 46 in Kontakt miteinander. Die Führungsflanke 46 des Rastelements 43 lenkt das Gegenrastelement 40 radial nach innen aus, so dass die Raststellungen einfach erreicht werden können. Bei einer Drehung des Bedienelements 6 in einer der ersten Drehrichtung entgegengerichteten zweiten Drehrichtung kommen die steilen Rastflanken 45 miteinander in Kontakt. Die Feder 23 ist vorteilhaft so ausgelegt, dass die Kraft der Feder 23 nicht ausreicht, um die Rastflanken 45 zu überwinden, so dass sich das Injektionsgerät 1 beim Loslassen des Bedienelements 6 zwischen zwei Raststellungen immer bis zur nächstniedrigen Raststellung zurückstellt. Auch eine symmetrische Auslegung der Rastelemente 40 und/oder 43 kann jedoch vorteilhaft sein. Die Rastflankenkönnen so ausgelegt sein, dass der Bediener eine einmal erreichte Raststellung überwinden und das Bedienelement 6 zu einer niedrigeren eingestellten Dosis zurück stellen kann. Die Rastflanken können auch so ausgelegt sein, dass das nicht möglich ist.

Fig. 11 zeigt eine Öffnung 94 am Mitnehmer 14, in der das zweite Ende 85 der Feder 23 (Fig. 8) eingehängt ist. Die Figuren 12 und 13 zeigen die Gestaltung des Rastarms 39, der das Gegenrastelement 40 trägt. Wie Fig. 13 auch zeigt, begrenzt der Rand 48 die Umfangsnut 63, in die der Halterand 64 der Injektionshülse 17 (Fig. 2) ragt.

Die Figuren 16 bis 19 zeigen das Rastteil 15. Wie insbesondere Fig. 19 zeigt, besitzt das Rastteil 19 zwei einander gegenüberliegend angeordnete Längsnuten 37 am Außenumfang zur drehfesten Verbindung mit der Injektionshülse 17. Am Rastteil 15 sind im Ausführungsbeispiel vier Rastelemente 41, 42, 43 und 44 ausgebildet, die identisch gestaltet und in unterschiedlichen Abständen zueinander am Umfang verteilt angeordnet sind. Das Rastelement 41 entspricht der Nullstellung, das Rastelement 42 ist der Priming-Stellung zugeordnet, das Rastelement 43 ist einer ersten Dosis und das Rastelement 44 einer zweiten Dosis zugeordnet. An seiner distalen Stirnseite 49 ist das Rastteil 15 abgerundet ausgebildet. Mit dieser Stirnseite 49 liegt das Rastteil 15 am Bedienelement 6 an. Durch die abgerundete Gestaltung ergeben sich bei der Drehung des Bedienelements 6 gegenüber dem Rastteil 15 geringe Reibkräfte.

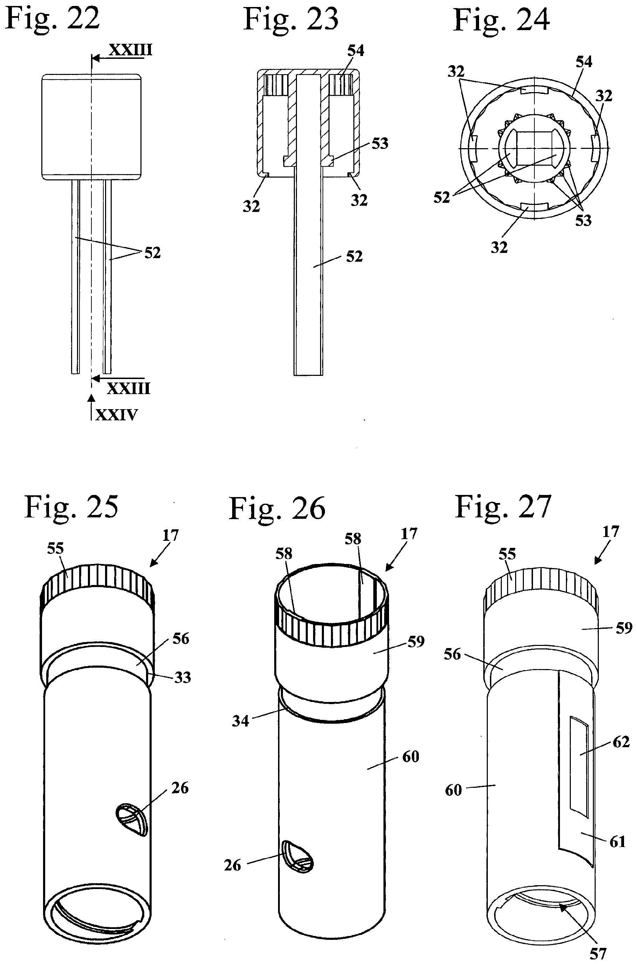

Die Figuren 20 bis 24 zeigen das Bedienelement 6. Wie die Figuren 20 und 21 zeigen, ist der Mitnahmeabschnitt 51 des Bedienelements 6, über den das Bedienelement 6 drehfest mit der Kolbenstange 12 verbunden ist (Fig. 1), durch zwei Arme 52 gebildet, die sich in Längsrichtung des Injektionsgeräts 1 erstrecken. Wie Fig. 23 zeigt, trägt das Bedienelement 6 im distalen Bereich seiner Umfangswand eine innenliegende Verzahnung 54, die mit einer in Fig. 25 gezeigten Verzahnung 55 am Außenumfang der Injektionshülse 17 zusammenwirkt und mit dieser die zweite Kupplung 20 bildet. Wie Fig. 24 zeigt, sind die Rastzähne 53, die mit den Rastzähnen 38 des Mitnehmers 14 die erste Kupplung 16 bilden, ebenfalls nicht über den gesamten Umfang des Bedienelements 6 verteilt, sondern jeweils nur in Teilbereichen angeordnet. Die Rastzähne 53 und die Rastzähne 38 sind so anzuordnen, dass in jeder Drehlage des Bedienelements 6 zum Mitnehmer 14 sichergestellt ist, dass mindestens ein Rastzahn 50 mit mindestens einem Rastzahn 38 in Eingriff kommt. Wie die Figuren 23 und 24 zeigen, ist das Anschlagelement 32 als nach innen ragender Rand ausgebildet. Im Ausführungsbeispiel ist kein durchgehender Rand vorgesehen, sondern es sind vier einzelne, voneinander getrennte Randabschnitte vorgesehen, die Anschlagelemente 32 bilden. Bei der Montage des Bedienelements schnappen die Anschlagelemente 32 hinter dem ersten Anschlag 33 der Injektionshülse 17 ein. Auch ein durchgehendes Anschlagelement 32, das sich über den gesamten Umfang erstreckt, kann jedoch vorteilhaft sein.

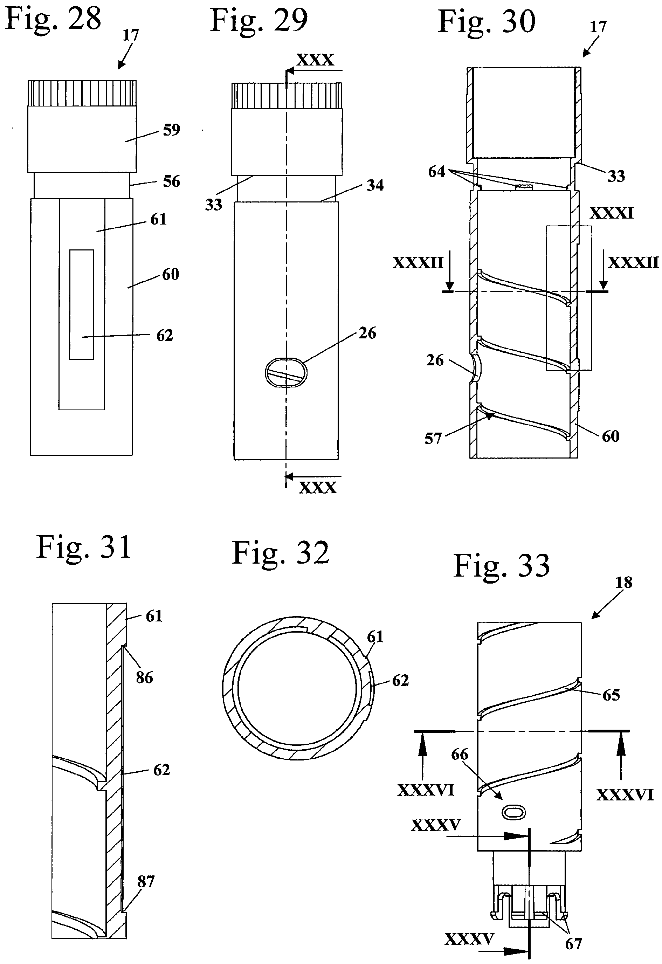

Die Figuren 25 bis 32 zeigen die Injektionshülse 17. Die Injektionshülse 17 ist hülsenförmig ausgebildet und besitzt einen distalen Abschnitt 59 und einen proximalen Abschnitt 60, die durch eine Nut 56 voneinander getrennt sind. An den die Nut 56 begrenzenden Seiten sind die Anschläge 33 und 34 ausgebildet. In die Nut 56 ragen die Anschlagelemente 32 des Bedienelements 6. Die Injektionshülse 17 besitzt an ihrer Innenseite zwei in Fig. 26 gezeigte Längsstege 58, die zur drehfesten Verbindung mit dem Rastteil 15 dienen und die in die Längsnuten 37 des Rastteils 15 ragen. In den Figuren 25 und 26 ist auch die Öffnung 26 gezeigt, durch die die Außenseite des Dosierorgans 18 für den Bediener sichtbar ist. Wie die Figuren 28 und 30 bis 32 zeigen, besitzt der proximale Abschnitt 60 der Injektionshülse 17 an der der Öffnung 26 gegenüberliegenden Seite einen Längssteg 61, der als Erhebung am Außenumfang ausgebildet ist. Der Längssteg 61 weist eine rechteckige Vertiefung 62 auf. Die Gestaltung der Vertiefung 62 ist in Fig. 31 im Einzelnen gezeigt.

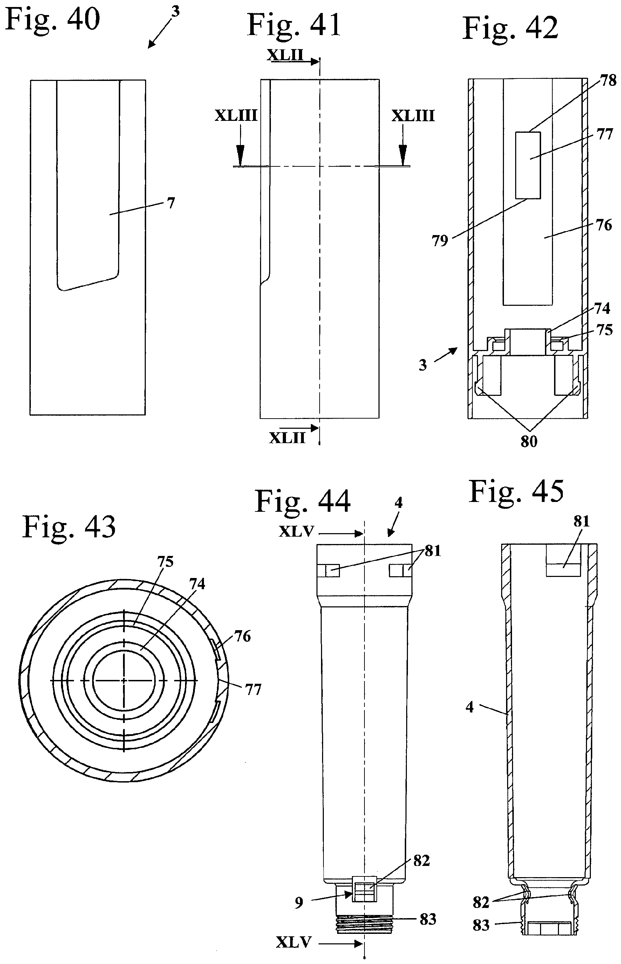

Die Vertiefung 62 besitzt eine distale Kante 86, die in Nullstellung 28 des Injektionsgeräts 1 mit einer in Fig. 42 gezeigten distalen Kante 78 am oberen Gehäuseteil 3 zusammenwirkt und mit dieser den ersten Anschlag 24 bildet. Die Vertiefung 62 besitzt eine proximale Kante 87, die in der in Fig. 4 gezeigten Injektionsstellung 29, die der maximal einstellbaren Menge an auszupressender Injektionsflüssigkeit entspricht, an einer proximalen Kante 79 des oberen Gehäuseteils 3 (Fig. 42) anliegt und mit dieser den zweiten Anschlag 25 bildet. Der zweite Anschlag 25 begrenzt die maximal einstellbare Menge an auszupressender Injektionsflüssigkeit.

Wie die Figuren 42 und 43 zeigen, besitzt das obere Gehäuseteil 3 eine Vertiefung 76, die als etwa rechteckförmige Längsnut ausgebildet ist und aus der sich ein Längssteg 77 erhebt. In die Vertiefung 76 ragt der Längssteg 61 der Injektionshülse 17. Dadurch ist die Injektionshülse 17 in Umfangsrichtung gegenüber dem Gehäuse 2 verdrehgesichert. Der Längssteg 77 des oberen Gehäuseteils 3 ragt in die Vertiefung 62 der Injektionshülse 17 und bildet mit dieser die Anschläge 24 und 25. Auch der Längssteg 77 und die Vertiefung 62 bilden eine Verdrehsicherung für die Injektionshülse 17. Wie insbesondere Fig. 30 zeigt, trägt die Injektionshülse 17 in ihrem proximalen Abschnitt 60 ein Innengewinde 57.

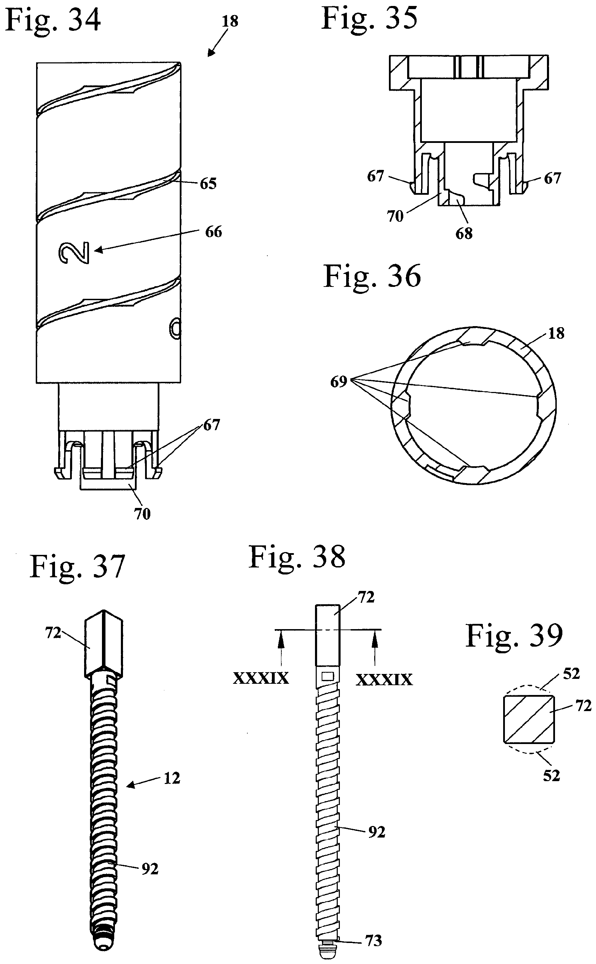

Die Figuren 33 bis 36 zeigen das Dosierorgan 18 im Einzelnen. Das Dosierorgan 18 trägt an seiner Außenseite ein Außengewinde 65, das mit dem Innengewinde 57 der Injektionshülse 17 (Fig. 30) die erste Gewindeverbindung 19 bildet. Im Bereich des Außengewindes 65 besitzt das Dosierorgan 18 die in den Figuren 33 und 34 gezeigte Skala 66, die dem Bediener die eingestellte Menge an Injektionsflüssigkeit anzeigt. Wie die Figuren 33 bis 35 zeigen, besitzt das Dosierorgan 18 an seiner proximalen Seite Rasthaken 67. Wie Fig. 42 zeigt, besitzt das obere Gehäuseteil 3 einen umlaufenden Rastrand 75, an dem die Rasthaken 67 einhaken und so die Verrastung 71 bilden. Wie die Figuren 34 und 35 zeigen, besitzt das Dosierorgan 18 an seiner proximalen Seite einen Lagerzapfen 70, der in einer Lagerhülse 74 des oberen Gehäuseteils 3 (Fig. 42) gelagert ist. Dadurch ist das Dosierorgan 18 im oberen Gehäuseteil 3 drehbar gelagert. Wie Fig. 35 zeigt, ist im Lagerzapfen 70 ein Innengewinde 68 angeordnet. Das Innengewinde 68 wirkt mit dem in Fig. 37 und Fig. 38 gezeigten Außengewinde 92 der Kolbenstange 12 zusammen und bildet mit diesem die zweite Gewindeverbindung 22. Wie Fig. 36 zeigt, besitzt das im Wesentlichen hülsenförmige Dosierorgan 18 an seinem Innenumfang insgesamt vier Längsstege 69, die zur drehfesten Verbindung mit dem Mitnehmer 14 dienen. Hierzu ragen die Längsstege 69 in die in den Figuren 11 bis 13 gezeigten Längsnuten 36 des Mitnehmers 14.

Wie die Figuren 37 bis 39 zeigen, besitzt die Kolbenstange 12 an ihrer distalen Seite einen Führungsabschnitt 72, der einen rechteckigen, im Ausführungsbeispiel einen quadratischen Querschnitt besitzt. Die in Fig. 39 schematisch eingezeichneten Arme 52 des Bedienelements 6 liegen an gegenüberliegenden Längsseiten des Führungsabschnitts 72 an und stellen dadurch eine drehfeste Verbindung zwischen Bedienelement 6 und Kolbenstange 12 her. An ihrem proximalen Bereich besitzt die Kolbenstange 12 eine Nut 73, an der die Kolbenscheibe 13 (Fig. 2) eingehängt ist.

Wie Fig. 42 zeigt, besitzt das obere Gehäuseteil 3 in seinem proximalen Bereich Rasthaken 80, die zur Verrastung mit dem in den Figuren 44 und 45 gezeigten Halter 4 dienen. Der Halter 4 besitzt in seinem distalen Bereich Rastöffnungen 81, in denen die Rasthaken 80 einhaken. Im Ausführungsbeispiel sind zwei Rasthaken 80 und zwei Rastöffnungen 81 gezeigt. In den Figuren 44 und 45 ist auch die Rasteinrichtung 9 gezeigt. Die Rasteinrichtung 9 wird durch zwei einander gegenüberliegende Rasthaken 82 gebildet, die am Behälter 5 einrasten. An seinem Außenumfang besitzt der Halter 4 im proximalen Bereich ein Außengewinde 83, auf das die Injektionsnadel 8 (Fig. 1) aufgeschraubt ist.

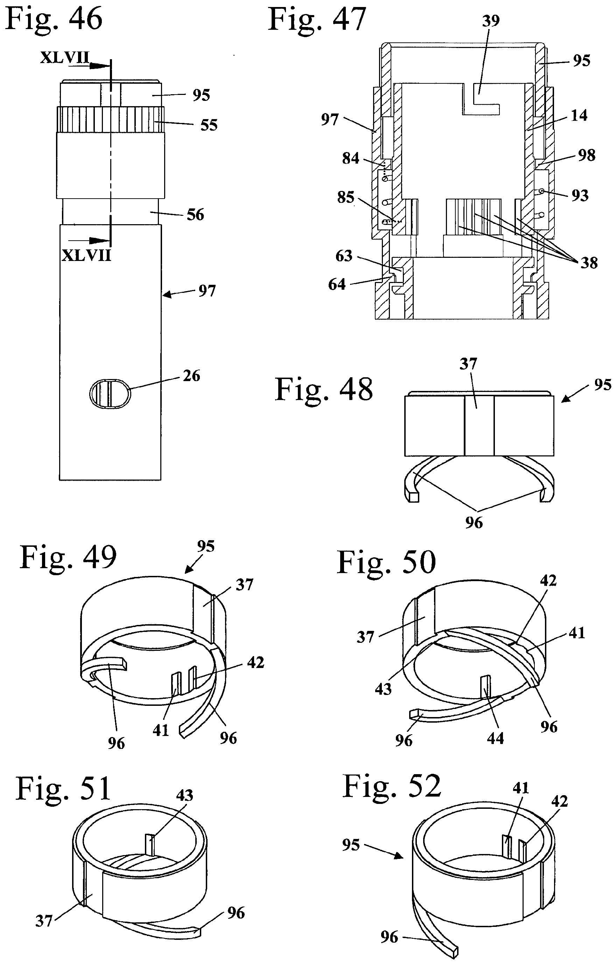

Im Ausführungsbeispiel nach den Figuren 1 bis 45 erfüllt die Feder 23 eine Doppelfunktion, da sie sowohl ein Drehmoment zwischen dem Mitnehmer 14 und dem Rastteil 15 aufbringen, als auch das Rastteil 15 und das Bedienelement 6 in distale Richtung vorspannen muss. Um die Auslegung der Feder 23 zu vereinfachen, können separate Federelemente zur Aufbringung der axialen Kraft und zur Aufbringung des Drehmoments vorgesehen werden. Eine entsprechende Ausführung ist in den Figuren 46 bis 52 gezeigt. Gleiche Bezugszeichen bezeichnen in allen Figuren der vorliegenden Anmeldung dabei einander entsprechende Elemente. Die Figuren 46 und 47 zeigen eine Injektionshülse 97, in der ein Rastteil 95 und ein Mitnehmer 14 angeordnet sind. Die Injektionshülse 97 besitzt einen nach innen ragenden Abstützrand 98, an dem ein erstes Ende 84 einer Feder 93 festgelegt ist. Ein zweites Ende 85 der Feder 93 ist am Mitnehmer 14 eingehängt. Die Feder 93 dient zur Aufbringung eines Drehmoments zwischen der Injektionshülse 97 und dem Mitnehmer 14. Da der Mitnehmer 14 drehfest mit dem Dosierorgan 18 verbunden ist, wirkt das Drehmoment zwischen Dosierorgan 18 und Injektionshülse 17 und spannt das Dosierorgan 18 in Richtung auf die Nullstellung 28 der Anordnung vor.

Wie die Figuren 48 bis 52 zeigen, besitzt das Rastteil 95 an seiner proximalen Seite zwei Federarme 96. Im Ausführungsbeispiel sind die Federarme 96 einteilig mit dem Rastteil 95 ausgebildet, vorzugsweise aus Kunststoff. Die Federarme 96 spannen das Rastteil 95 aufgrund ihrer Eigenelastizität in die distale Richtung vor. Anstatt der Federarme 96 kann auch eine Schraubendruckfeder oder ein anders gestaltetes Federelement zur Vorspannung des Rastteils 95 und des Bedienelements 6 in Richtung der Längsmittelachse 50 zweckmäßig sein.

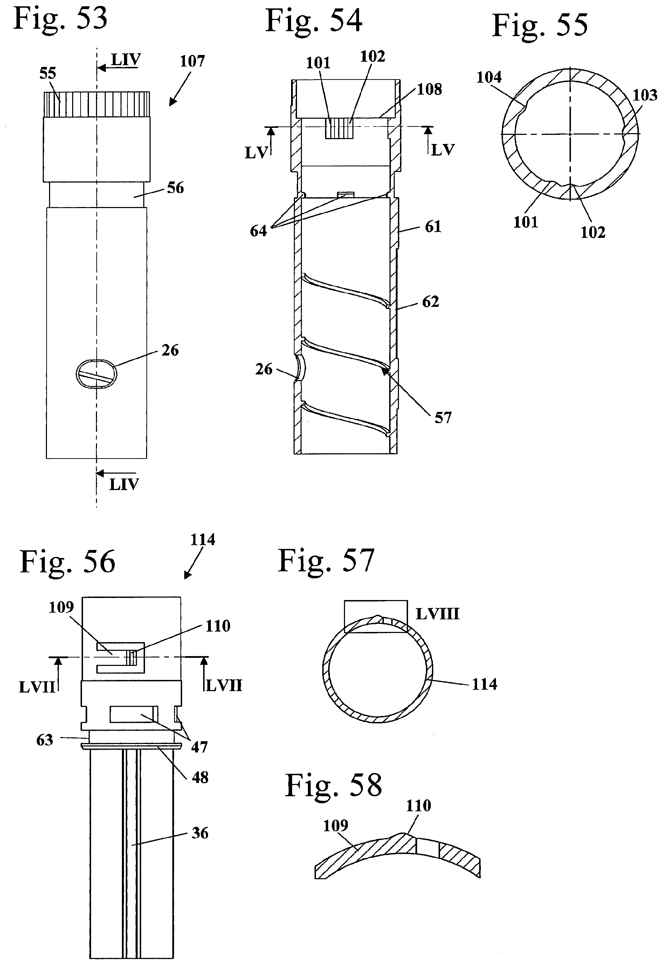

Die Figuren 53 bis 58 zeigen ein weiteres Ausführungsbeispiel eines Injektionsgeräts, wobei lediglich die Injektionshülse 107 und der Mitnehmer 114 gezeigt sind. Die weiteren, nicht gezeigten Elemente entsprechen den zum Injektionsgerät 1 gezeigten und beschriebenen Bauteilen. Wie Fig. 54 zeigt, besitzt die Injektionshülse 107 an ihrem Innenumfang Rastelemente 101, 102, 103 und 104. Die Rastelemente 101, 102, 103 und 104 legen die Rastpositionen des Injektionsgeräts 1 fest. Wie Fig. 54 zeigt, besitzen die Rastelemente 101 bis 104 am Umfang unterschiedliche Abstände zueinander. Das Rastelement 101 ist der Nullstellung zugeordnet, das Rastelement 102 der Priming-Stellung und die Rastelemente 103 und 104 einer ersten und zweiten Menge an auszupressender Injektionsflüssigkeit.

Wie die Figuren 56 bis 58 zeigen, besitzt der Mitnehmer 114 einen Rastarm 109, an dem ein Gegenrastelement 110 ausgebildet ist, das mit den Rastelementen 101 bis 104 zur Definition der Raststellungen zusammenwirken kann. Da die relative Lage von Injektionshülse 107 und Mitnehmer 114 aufgrund der in die Umfangsnut 63 ragenden Halteränder 64 vorgegeben ist, ist die durch die Rastelemente 101 bis 104 und das Gegenrastelement 110 gebildete Rasteinrichtung sowohl beim Einstellen einer auszupressenden Menge an Injektionsflüssigkeit als auch beim Auspressen der Injektionsflüssigkeit aus dem Behälter wirksam. Wie die Figuren 55 und 58 zeigen, sind die Rastelemente 101 bis 104 und das Gegenrastelement 110 in Umfangsrichtung etwa symmetrisch ausgebildet, so dass beim Auspressen der Injektionsflüssigkeit keine zu hohe Kraft zum Überwinden der Raststellungen benötigt wird. Bei dem in den Figuren 53 bis 58 gezeigten Injektionsgerät ist keine Feder vorgesehen, die das Dosierorgan in Richtung auf die Nullstellung vorspannt. Am Innenumfang der Injektionshülse 107 ist ein Absatz 108 zur Auflage einer Druckfeder vorgesehen, die zwischen der Injektionshülse 107 und einem in den Figuren 53 bis 58 nicht gezeigten Bedienelement wirkt und das Bedienelement in distale Richtung vorspannt.

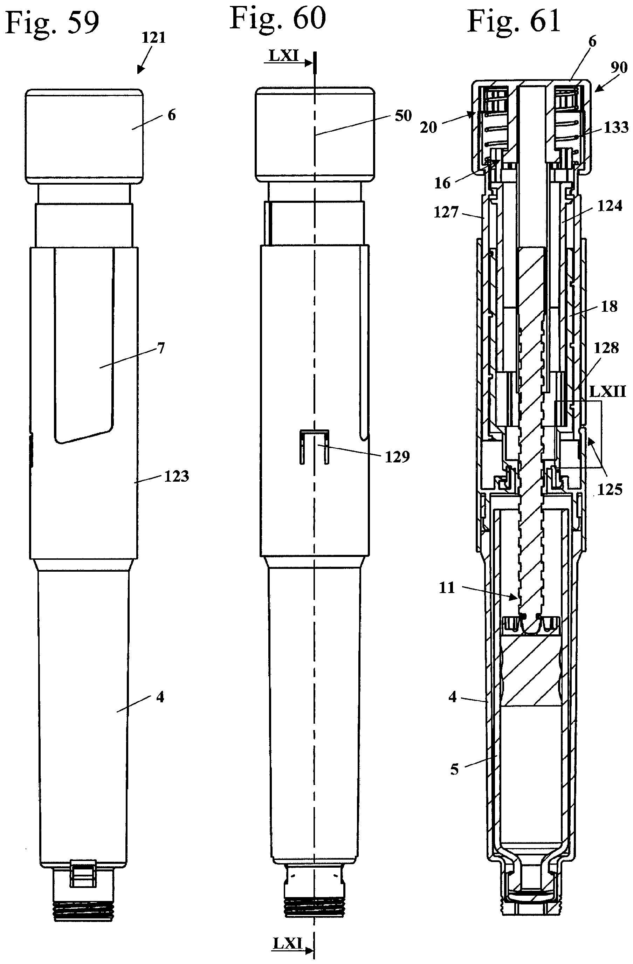

Ein weiteres Ausführungsbeispiel eines Injektionsgeräts 121 ist in den Figuren 59 bis 62 gezeigt. Das Injektionsgerät 121 besitzt ein oberes Gehäuseteil 123, an dem ein Rastarm 129 ausgebildet ist. Im Ausführungsbeispiel ist der Rastarm 129 von außen sichtbar. Vorteilhaft ist der Rastarm 129 jedoch so ausgebildet, dass er für den Bediener nicht sichtbar ist. Das Injektionsgerät 121 besitzt eine Injektionshülse 127, die im Wesentlichen der Injektionshülse 17 des Injektionsgeräts 1 entspricht. Die Injektionshülse 127 besitzt jedoch an ihrer Außenseite Rastelemente 128, die als Vertiefungen ausgebildet sind und in die ein am Rastarm 129 ausgebildetes Gegenrastelement 130, das im Ausführungsbeispiel als Raste ausgebildet ist, einrasten kann und mit diesen eine Rasteinrichtung 125 bildet. Wie Fig. 61 zeigt, besitzt das Injektionsgerät 121 einen Mitnehmer 124, der keine Rastelemente oder Gegenrastelemente trägt. Auch ein Rastteil ist nicht vorgesehen. Im Bedienelement 6 ist eine Feder 133 angeordnet, die das Bedienelement 6 in distale Richtung vorspannt. Im Ausführungsbeispiel wirkt die Rasteinrichtung 125 zwischen dem oberen Gehäuseteil 123 und der Injektionshülse 127 sowohl beim Einstellen einer auszupressenden Menge an Injektionsflüssigkeit als auch beim Auspressen der Injektionsflüssigkeit aus dem Behälter 5. Es könnte jedoch auch vorgesehen sein, mit dem Verstellen des Bedienelements 6 in seine proximale Stellung 91 (Fig. 6) den Rastarm 129 so auszulenken, dass das Gegenrastelement 130 nicht mit Rastelementen 128 zusammenwirken kann. Dies ist insbesondere dann zweckmäßig, wenn der Rastarm 129 nicht am oberen Gehäuseteil 123, sondern an der Injektionshülse 127 angeordnet wird und die Rastelemente 128 am oberen Gehäuseteil 123.

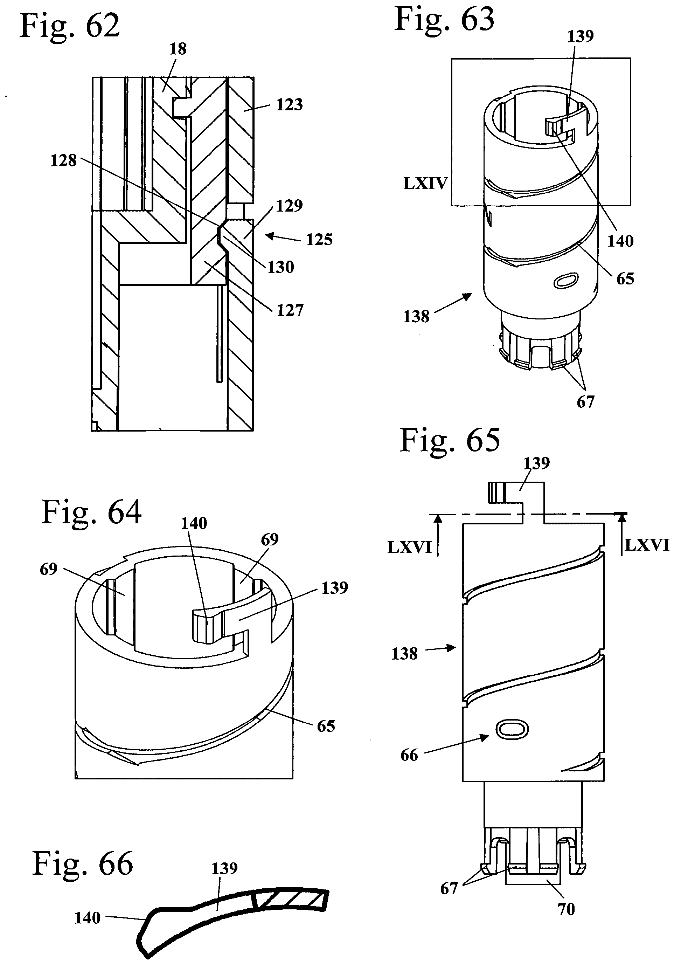

Bei dem in den Figuren 59 bis 62 gezeigten Injektionsgerät 121 kann auch eine Feder vorgesehen sein, die das Dosierorgan in der zweiten Drehrichtung vorspannt und die zwischen Injektionshülse 127 und Dosierorgan wirkt. Wie Fig. 62 zeigt, sind das Rastelement 128 und das Gegenrastelement 130 in der gezeigten Ausführung symmetrisch ausgeführt. Wird zusätzlich eine Feder zur Vorspannung des Dosierorgans in der zweiten Drehrichtung vorgesehen, kann es zweckmäßig sein, die Rastelemente unsymmetrisch auszubilden, so dass sich unterschiedliche Kräfte zum Überwinden der Raststellungen beim Einstellen und beim Auspressen der Injektionsflüssigkeit ergeben und die zusätzliche Feder das Dosierorgan jeweils nur bis zur nächstniedrigen vorgesehenen Menge an Injektionsflüssigkeit zurückdrehen kann.

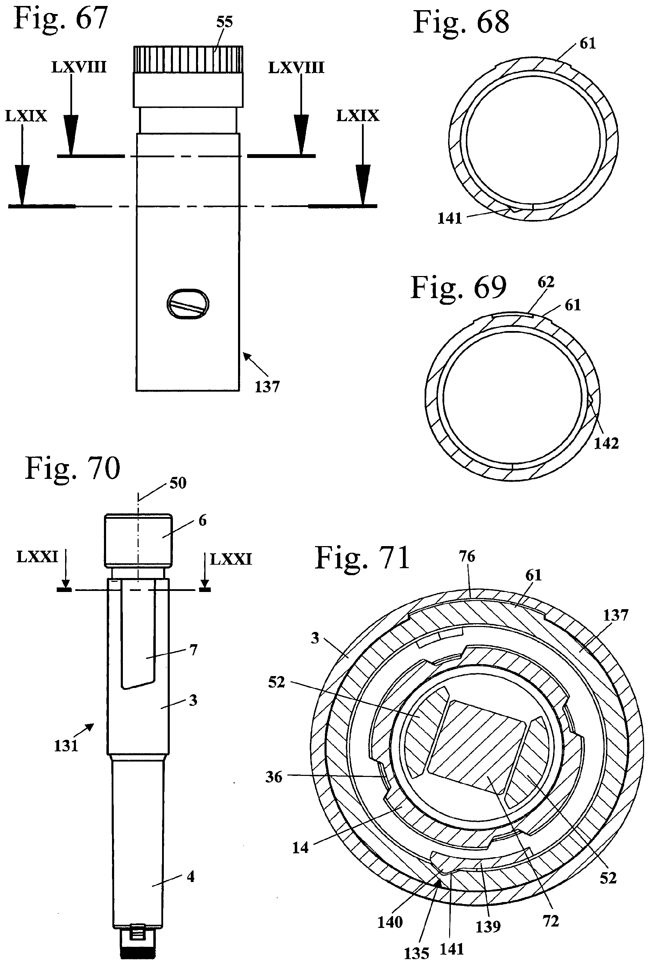

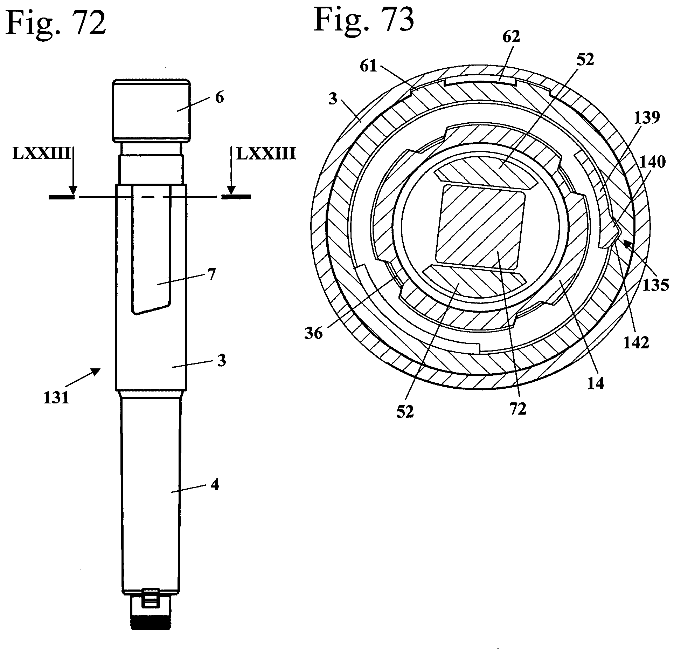

Die Figuren 63 bis 73 zeigen ein weiteres Ausführungsbeispiel eines Injektionsgeräts 131 (Fig. 70 und 72). Die Figuren 63 bis 66 zeigen das Dosierorgan 138 des Injektionsgeräts 131. Das Dosierorgan 138 besitzt an seiner distalen Seite einen Rastarm 139, der ein Gegenrastelement 140 trägt. Der weitere Aufbau des Dosierorgans 138 entspricht im Wesentlichen dem Aufbau des Dosierorgans 18. Wie Fig. 66 zeigt, ist das Gegenrastelement 140 im Ausführungsbeispiel symmetrisch ausgebildet. Wie die Figuren 67 bis 69 zeigen, besitzt das Injektionsgerät 131 eine Injektionshülse 137, die an ihrem Innenumfang Rastelemente 141 und 142 trägt. Im Ausführungsbeispiel sind die Rastelemente 141 und 142 als Vertiefungen ausgebildet. Es können weitere Rastelemente vorgesehen sein. Es kann vorteilhaft sein, den Rastarm 139 statt am Dosierorgan 138 an der Injektionshülse 137 auszubilden und entsprechende Rastelemente oder Rastvertiefungen am Dosierorgan 138 vorzusehen. Die Rastelemente 141 und 142 liegen zueinander sowohl in Richtung der Längsmittelachse 50 als auch in Umfangsrichtung versetzt. Die Rastelemente 141 und 142 liegen auf einer schraubenlinienförmigen Bahn, die dem Gewindegang des Außengewindes 65 des Dosierorgans 138 entspricht. Dadurch, dass die Rastelemente 141 und 142 sowohl in Richtung der Längsmittelachse 50 als auch in Umfangsrichtung zueinander versetzt sind, sind vergleichsweise geringe Abstände zwischen den Raststellungen möglich.

Die Figuren 70 und 71 zeigen das Injektionsgerät 131 in Nullstellung. Das Rastelement 141 ist im Gegenrastelement 140 eingerastet und bildet mit diesem eine Rasteinrichtung 135. In den Figuren 72 und 73 befindet sich das Injektionsgerät 131 in einer Injektionsstellung. Das Gegenrastelement 140 ist am Rastelement 142 eingerastet. Aufgrund der symmetrischen Ausbildung der Rastelemente 141, 142 und des Gegenrastelements 140 können die Raststellungen vom Bediener leicht in beide Drehrichtungen überdrückt werden, so dass der Bediener das Injektionsgerät 131 von einer einmal eingestellten Dosis bis zur Nullstellung zurückstellen kann, ohne dass Injektionsflüssigkeit ausgepresst wird. Es kann jedoch auch eine andere, unsymmetrische Auslegung der Rastelemente zweckmäßig sein. Auch bei den in den Figuren 63 bis 73 gezeigten Ausführungsbeispielen eines Injektionsgeräts 131 kann eine Feder zwischen der Injektionshülsel37 und dem Dosierorgan 138 vorgesehen sein, die das Dosierorgan 138 in der zweiten Drehrichtung vorspannt. Da sich die Injektionshülse 137 gegenüber dem Dosierorgan 138 nicht nur in Umfangsrichtung, sondern auch in axialer Richtung bewegt, ist auch bei mehreren Umdrehungen des Bedienelements 6 bis zur Maximaldosis jeder relativen Lage von Injektionshülse 137 und Dosierorgan 138 eindeutig eine eingestellte Menge an Injektionsflüssigkeit zugeordnet.

## Patentansprüche

1. Injektionsgerät mit einem Gehäuse (2), mit einer Längsmittelachse (50), mit einem drehbar und in Richtung der Längsmittelachse (50) fest im Gehäuse (2) gehaltenen Dosierorgan (18, 138), das über eine erste Gewindeverbindung (19) mit einer gegenüber dem Gehäuse (2) drehfest und in Richtung der Längsmittelachse (50) verschiebbar gehaltenen Injektionshülse (17, 97, 107, 127, 137) verbunden ist,
wobei sich das Dosierorgan (18, 138) beim Einstellen einer auszupressenden Menge an Injektionsflüssigkeit in einer ersten Drehrichtung gegenüber dem Gehäuse (2) dreht und sich die Injektionshülse (17, 97, 107, 127, 137) aufgrund der ersten Gewindeverbindung (19) in distale Richtung (30) bewegt, und wobei sich das Dosierorgan (18, 138) beim Auspressen einer eingestellten Menge an Injektionsflüssigkeit in einer der ersten Drehrichtung entgegengerichteten zweiten Drehrichtung gegenüber dem Gehäuse (2) dreht und sich die Injektionshülse (17, 97, 107, 127, 137) aufgrund der ersten Gewindeverbindung (19) in proximale Richtung (31) bewegt, mit einem Dosierkolben (11) zum Auspressen von Injektionsflüssigkeit aus einem Behälter (5), wobei der Dosierkolben (11) über eine zweite Gewindeverbindung (22) mit dem Dosierorgan (18, 138) verbunden ist, wobei der Dosierkolben (11) beim Einstellen der auszupressenden Menge an Injektionsflüssigkeit drehfest mit dem Dosierorgan (18, 138) verbunden ist und sich mit dem Dosierorgan (18, 138) dreht, und wobei der Dosierkolben (11) beim Auspressen einer auszupressenden Menge an Injektionsflüssigkeit drehfest mit der Injektionshülse (17, 97, 107, 127, 137) verbunden ist und sich aufgrund der zweiten Gewindeverbindung (22) in proximale Richtung (31) bewegt, und mit einer Rasteinrichtung (35, 125, 135), die mindestens beim Einstellen der aus dem Behälter (5) auszupressenden Menge an Injektionsflüssigkeit wirksam ist, **dadurch gekennzeichnet, dass** die Rasteinrichtung (35, 125, 135) zwischen zwei Bauteilen des Injektionsgeräts (1, 121, 131) wirkt, die sich beim Einstellen der auszupressenden Menge an Injektionsflüssigkeit relativ zueinander bewegen, wobei jeder relativen Lage der beiden Bauteile zueinander eindeutig eine eingestellte Menge an Injektionsflüssigkeit zugeordnet ist.

2. Injektionsgerät nach Anspruch 1,
**dadurch gekennzeichnet, dass** die Rasteinrichtung (35, 125, 135) mindestens ein Rastelement (41, 42, 43, 44, 101, 102, 103, 104, 128, 141, 142) und mindestens ein mit dem Rastelement (41, 42, 43, 44, 101, 102, 103, 104, 128, 141, 142) in einer Raststellung zusammenwirkendes Gegenrastelement (40, 110, 130, 140) aufweist.

3. Injektionsgerät nach Anspruch 1 oder 2,
**dadurch gekennzeichnet, dass** eines der beiden Bauteile, zwischen denen die Rasteinrichtung (135) wirkt, das Dosierorgan (138) und das andere der beiden Bauteile die Injektionshülse (137) ist.

4. Injektionsgerät nach Anspruch 1 oder 2,
**dadurch gekennzeichnet, dass** eines der beiden Bauteile, zwischen denen die Rasteinrichtung (125) wirkt, die Injektionshülse (127) und das andere der beiden Bauteile das Gehäuse (2) ist

5. Injektionsgerät nach einem der Ansprüche 1 bis 4,
**dadurch gekennzeichnet, dass** das Injektionsgerät (1, 121, 131) ein Bedienelement (6) besitzt, das über eine erste Kupplung (16) drehfest mit einem drehfest mit dem Dosierorgan (18, 138) verbundenen Mitnehmer (14, 114, 124) und über eine zweite Kupplung (20) mit der Injektionshülse (17, 97, 107, 127, 137) verbindbar ist, wobei das Bedienelement (6) bezogen auf die Injektionshülse (17, 97, 107, 127, 137) eine distale Stellung (90) und eine proximale Stellung (91) besitzt, wobei das Bedienelement (6) in der distalen Stellung (90) über die erste Kupplung (16) drehfest mit dem Mitnehmer (14, 114, 124) verbunden ist und die zweite Kupplung (20) geöffnet ist, so dass das Bedienelement (6) gegenüber der Injektionshülse (17, 97, 107, 127, 137) drehbar ist, und wobei in der proximalen Stellung (91) die erste Kupplung (16) geöffnet und das Bedienelement (6) gegenüber dem Mitnehmer (14, 114, 124) drehbar ist und das Bedienelement (6) über die zweite Kupplung (20) drehfest mit der Injektionshülse (17, 97, 107, 127, 137) verbunden ist.

6. Injektionsgerät nach Anspruch 5,
**dadurch gekennzeichnet, dass** die Rasteinrichtung (35) in der distalen Stellung (90) des Bedienelements (6) wirksam ist und in der proximalen Stellung (91) des Bedienelements (6) das mindestens eine Rastelement (41, 42, 43, 44) und das mindestens eine Gegenrastelement (40) unabhängig von der relativen Lage der beiden Bauteile zueinander außer Eingriff sind.

7. Injektionsgerät nach Anspruch 5 oder 6,
**dadurch gekennzeichnet, dass** eines der beiden Bauteile, zwischen denen die Rasteinrichtung (35) wirkt, der Mitnehmer (14, 114) und das andere der beiden Bauteile die Injektionshülse (17, 97, 107) ist.

8. Injektionsgerät nach einem der Ansprüche 5 bis 7,
**dadurch gekennzeichnet, dass** der Mitnehmer (14, 114, 124) in Richtung der Längsmittelachse (50) an die Stellung der Injektionshülse (17, 97, 107, 127, 137) gekoppelt ist.

9. Injektionsgerät nach einem der Ansprüche 2 bis 8,
**dadurch gekennzeichnet, dass** mindestens ein Rastelement (101, 102, 103, 104, 128, 141, 142) an einem der beiden Bauteile, zwischen denen die Rasteinrichtung (125, 135) wirkt, angeordnet ist und mindestens ein Gegenrastelement (110, 130, 140) an dem anderen der beiden Bauteile.

10. Injektionsgerät nach einem der Ansprüche 2 bis 8,
**dadurch gekennzeichnet, dass** mindestens ein Rastelement (41, 42, 43, 44) an einem Rastteil (15, 95) angeordnet ist, das mit dem einen der beiden Bauteile, zwischen denen die Rasteinrichtung (35) wirkt, drehfest und in Richtung der Längsmittelachse (50) gegenüber diesem Bauteil verschiebbar verbunden ist, wobei das mindestens eine Rastelement (41, 42, 43, 44) in einer ersten axialen Stellung (88) des Rastteils (15) in Eingriff mit dem mindestens einen Gegenrastelement (40) kommen kann und in einer zweiten axialen Stellung (89) des Rastteils (15) unabhängig von der relativen Lage der beiden Bauteile zueinander außer Eingriff mit dem mindestens einen Gegenrastelement (40) ist.

11. Inj ektionsgerät nach Anspruch 10,
**dadurch gekennzeichnet, dass** das Rastteil (15, 95) in Richtung auf seine erste axiale Stellung (88) vorgespannt ist.

12. Inj ektionsgerät nach Anspruch 11,
**dadurch gekennzeichnet, dass** das Rastteil (15, 95) mindestens einen Federarm (96) aufweist, der das Rastteil (15, 95) in Richtung auf seine erste axiale Stellung (88) vorspannt.

13. Injektionsgerät nach einem der Ansprüche 1 bis 12,
**dadurch gekennzeichnet, dass** zwischen der Injektionshülse (17, 97) und dem Dosierorgan (18) eine Feder (23, 93) wirkt, die das Dosierorgan (18) in der zweiten Drehrichtung vorspannt.

14. Injektionsgerät nach Anspruch 13,
**dadurch gekennzeichnet, dass** die Feder (93) mit einem Ende (84) an der Injektionshülse (97) und mit dem anderen Ende (85) am Mitnehmer (14) festgelegt ist.

15. Injektionsgerät nach Anspruch 13,
**dadurch gekennzeichnet, dass** die Feder (23) mit einem Ende (84) am Rastteil (15) und mit dem anderen Ende (85) am Mitnehmer (14) festgelegt ist.

## Claims

1. Injection device with a housing (2), with a longitudinal center axis (50), and with a dosing member (18, 138), which is mounted rotatably and fixed in the direction of the longitudinal center axis (50) in the housing (2) and connected via a first threaded connection (19) to an injection sleeve (17, 97, 107, 127, 137) held non-rotatably relative to the housing (2) and displaceably in the direction of the longitudinal center axis (50),
wherein the dosing member (18, 138), when setting a quantity of injection fluid to be pressed out, rotates in a first direction of rotation relative to the housing (2) and the injection sleeve (17, 97, 107, 127, 137) moves in the distal direction (30) because of the first threaded connection (19), and wherein the dosing member (18, 138), when pressing out a set quantity of injection fluid, rotates relative to the housing (2) in a second direction of rotation opposed to the first direction of rotation and the injection sleeve (17, 97, 107, 127, 137) moves in the proximal direction (31) because of the first threaded connection (19), with a metering piston (11) for pressing out injection fluid from a container (5), wherein the metering piston (11) is connected to the dosing member (18, 138) via a second threaded connection (22), wherein the dosing member (11), when setting the quantity of injection fluid to be pressed out, is non-rotatably connected to the dosing member (18, 138) and rotates with the dosing member (18, 138), and wherein the metering piston (11), when pressing out a set quantity of injection fluid, is non-rotatably connected to the injection sleeve (17, 97, 107, 127, 137) and moves in the proximal direction (31) because of the second threaded connection (22), and with a latching device (35, 125, 135), which is active at least when setting the quantity of injection fluid to be pressed out from the container (5),
**characterised in that** the latching device (35, 125, 135) acts between two components of the injection device (1, 121, 131) which move relative to each other when setting the quantity of injection fluid to be pressed out, wherein a set quantity of injection fluid is unequivocally assigned to each relative position of the two components.

2. Injection device according to claim 1,
**characterised in that** the latching device (35, 125, 135) comprises at least one latching element (41, 42, 43, 44, 101, 102, 103, 104, 128, 141, 142) and at least one counter-latching element (40, 110, 130, 140) acting together with the latching element (41, 42, 43, 44, 101, 102, 103, 104, 128, 141, 142) in a latching position.

3. Injection device according to claim 1 or 2,
**characterised in that** one of the two components between which the latching device (135) acts is the dosing member (138) and the other of the two components is the injection sleeve (137).

4. Injection device according to claim 1 or 2,
**characterised in that** one of the two components between which the latching device (125) acts is the injection sleeve (127) and the other of the two components is the housing (2).

5. Injection device according to any of claims 1 to 4,
**characterised in that** the injection device (1, 121, 131) has a control (6), which is non-rotatably connected to a driver (14, 114, 124) non-rotatably connected to the dosing member (18, 138) via a first clutch (16) and can be connected to the injection sleeve (17, 97, 107, 127, 137) via a second clutch (20), wherein the control (6) has a distal position (90) and a proximal position (91) relative to the injection sleeve (17, 97, 107, 127, 137), wherein the control (6) is non-rotatably connected to the driver (14, 114, 124) via the first clutch (16) in the distal position (90) and the second clutch (20) is open, so that the control (6) is rotatable relative to the injection sleeve (17, 97, 107, 127, 137), and wherein the first clutch (16) is open and the control (6) is rotatable relative to the driver (14, 114, 124) in the proximal position (91) and the control (6) is non-rotatably connected to the injection sleeve (17, 97, 107, 127, 137) via the second clutch (20).

6. Injection device according to claim 5,
**characterised in that** the latching device (35) is active in the distal position (90) of the control (6), and **in that** in the proximal position (91) of the control (6) the at least one latching element (41, 42, 43, 44) and the at least one counter-latching element (40) are out of engagement irrespective of the relative positions of the two components.

7. Injection device according to claim 5 or 6,
**characterised in that** one of the two components between which the latching device (35) acts is the driver (14, 114) and the other of the two components is the injection sleeve (17, 97, 107).

8. Injection device according to any of claims 5 to 7,
**characterised in that** the driver (14, 114, 124) is coupled to the position of the injection sleeve (17, 97, 107, 127, 137) in the direction of the longitudinal center axis (50).

9. Injection device according to any of claims 2 to 8,
**characterised in that** at least one latching element (101, 102, 103, 104, 128, 141, 142) is located on one of the two components between which the latching device (125, 135) acts and at least one counter-latching element (110, 130, 140) is located on the other of the two components.

10. Injection device according to any of claims 2 to 8,
**characterised in that** at least one latching element (41,42, 43, 44) is located on a latching part (15, 95), which is non-rotatably connected to one of the two components between which the latching device (35) acts and is displaceable relative to this component in the direction of the longitudinal center axis (50), wherein the at least one latching element (41,42, 43, 44) can come into engagement with the at least one counter-latching element (40) in a first axial position (88) of the latching part (15) and is out of engagement with the at least one counter-latching element (40) in a second axial position (89) of the latching part (15) irrespective of the relative positions of the two components.

11. Injection device according to claim 10,
**characterised in that** the latching part (15, 95) is biased towards its first axial position (88).

12. Injection device according to claim 11,
**characterised in that** the latching part (15, 95) has at least one spring arm (96), which biases the latching part (15, 95) towards its first axial position (88).

13. Injection device according to any of claims 1 to 12,
**characterised in that** a spring (23, 83) biasing the metering element (18) in the second direction of rotation acts between the injection sleeve (17, 97) and the dosing member (18).

14. Injection device according to claim 13,
**characterised in that** the spring (93) is fixed to the injection sleeve (97) at one end (84) and to the driver (14) at the other end (85).

15. Injection device according to claim 13,
**characterised in that** the spring (23) is fixed to the latching part (15) at one end (84) and to the driver (14) at the other end (85).

## Revendications

1. Appareil d'injection comprenant un boîtier (2), un axe médian longitudinal (50), un organe de dosage (18, 138) qui est maintenu de façon à pouvoir tourner et fixe en direction de l'axe médian longitudinal (50) dans le boîtier (2), qui est relié par un premier raccord fileté (19) à une gaine d'injection (17, 97, 107, 127, 137) maintenue de façon solidaire en rotation par rapport au boîtier (2) et de façon à pouvoir coulisser en direction de l'axe longitudinal (50),
dans lequel l'organe de dosage (18, 138) tourne lors du réglage d'une quantité à exprimer de fluide d'injection dans une première direction de rotation par rapport au boîtier (2) et la gaine d'injection (17, 97, 107, 127, 137) se déplace sur la base du premier raccord fileté (19) en direction distale (30), et dans lequel l'organe de dosage (18, 138) tourne lors de l'expression d'une quantité réglée de fluide d'injection dans une deuxième direction de rotation opposée à la première direction de rotation par rapport au boîtier (2) et la gaine d'injection (17, 97, 107, 127, 137) se déplace sur la base du premier raccord fileté (19) en direction proximale (31), avec un piston de dosage (11) pour l'expression de fluide d'injection hors d'un récipient (5), dans lequel le piston de dosage (11) est relié par un deuxième raccord fileté (22) avec l'organe de dosage (18, 138), dans lequel le piston de dosage (11) est relié de façon solidaire en rotation lors du réglage de la quantité à exprimer de fluide d'injection avec l'organe de dosage (18, 138) et tourne avec l'organe de dosage (18, 138), et dans lequel le piston de dosage (11) lors de l'expression d'une quantité à exprimer de fluide d'injection est relié de façon solidaire en rotation à la gaine d'injection (17, 97, 107, 127, 137) et se déplace sur la base du deuxième raccord fileté (22) en direction proximale (31), et avec un système de blocage (35, 125, 135) qui agit au moins lors du réglage de la quantité de fluide d'injection à exprimer hors du récipient (5), **caractérisé en ce que** le système de blocage (35, 125, 135) agit entre deux composants de l'appareil d'injection (1, 121, 131) qui se déplacent l'un par rapport à l'autre lors du réglage de la quantité à exprimer de fluide d'injection, dans lequel à chaque emplacement relatif des deux composants l'un par rapport à l'autre est affectée clairement une quantité réglée de fluide d'injection.

2. Appareil d'injection selon la revendication 1,
**caractérisé en ce que** le système de blocage (35, 125, 135) présente au moins un élément de blocage (41, 42, 43, 44, 101, 102, 103, 104, 128, 141, 142) et au moins élément de contre-blocage (40, 110, 130, 140) coopérant avec l'élément de blocage (41, 42, 43, 44, 101, 102, 103, 104, 128, 141, 142) dans une position de blocage.

3. Appareil d'injection selon la revendication 1 ou 2,
**caractérisé en ce qu'**un des deux composants, entre lesquels agit le système de blocage (135), est l'organe de dosage (138) et l'autre des deux composants est la gaine d'injection (137).

4. Appareil d'injection selon la revendication 1 ou 2,
**caractérisé en ce qu'**un des deux composants, entre lesquels agit le système de blocage (125), est la gaine d'injection (127) et l'autre des deux composants est le boîtier (2).

5. Appareil d'injection selon l'une quelconque des revendications 1 à 4,
**caractérisé en ce que** l'appareil d'injection (1, 121, 131) possède un élément de service (6), qui peut être relié par un premier couplage (16) de façon solidaire en rotation avec un élément d'entraînement (14, 114, 124) relié de façon solidaire en rotation avec l'organe de dosage (18, 138) et par un deuxième couplage (20) avec la gaine d'injection (17, 97, 107, 127, 137), dans lequel l'élément de service (6) possède par rapport à la gaine d'injection (17, 97, 107, 127, 137) une position distale (90) et une position proximale (91), dans lequel l'élément de service (6) dans la position distale (90) est relié par un premier couplage de façon solidaire en rotation (16) avec l'élément d'entraînement (14, 114, 124) et le deuxième couplage (20) est ouvert, de sorte que l'élément de service (6) peut tourner par rapport à la gaine d'injection (17, 97, 107, 127, 137), et dans lequel dans la position proximale (91), le premier couplage (16) est ouvert et l'élément de service (6) peut tourner par rapport à l'élément d'entraînement (14, 114, 124) et l'élément de service (6) est relié de façon solidaire en rotation par le deuxième couplage (20) avec la gaine d'injection (17, 97, 107, 127, 137).

6. Appareil d'injection selon la revendication 5,
**caractérisé en ce que** le système de blocage (35) est actif dans la position distale (90) de l'élément de service (6) et dans la position proximale (91) de l'élément de service (6), l'au moins un élément de blocage (41, 42, 43, 44) et l'au moins un élément de contre-blocage (40) sont hors de prise indépendamment de l'emplacement relatif des deux composants l'un par rapport à l'autre.

7. Appareil d'injection selon la revendication 5 ou 6,
**caractérisé en ce que** l'un des deux composants, entre lesquels agit le système de blocage (35), est l'élément d'entraînement (14, 114) et l'autre des deux composants est la gaine d'injection (17, 97, 107).

8. Appareil d'injection selon l'une quelconque des revendications 5 à 7,
**caractérisé en ce que** l'élément d'entraînement (14, 114, 124) est couplé en direction de l'axe médian longitudinal (50) à la position de la gaine d'injection (17, 97, 107, 127, 137).

9. Appareil d'injection selon l'une quelconque des revendications 2 à 8,
**caractérisé en ce que** l'au moins un élément de blocage (101, 102, 103, 104, 128, 141, 142) est disposé sur l'un des deux composants, entre lesquels le système de blocage (125, 135) agit, et au moins un élément de contre-blocage (110, 130, 140) sur l'autre des deux composants.

10. Appareil d'injection selon l'une quelconque des revendications 2 à 8,
**caractérisé en ce qu'**au moins un élément de blocage (41, 42, 43, 44) est disposé sur une partie de blocage (15, 95), qui est reliée de façon solidaire en rotation avec l'un des deux composants entre lesquels agit le système de blocage (35) et peut être décalé en direction de l'axe médian longitudinal (50) par rapport à ce composant, dans lequel l'au moins un élément de blocage (41, 42, 43, 44) peut venir en prise dans une première position axiale (88) de la partie de blocage (15) avec l'au moins un élément de contre-blocage (40) et dans une deuxième position axiale (89) de la partie de blocage (15) est hors de prise indépendamment de l'emplacement relatif des deux composants l'un par rapport à l'autre avec l'au moins un élément de contre-blocage (40).

11. Appareil d'injection selon la revendication 10,
**caractérisé en ce que** la partie de blocage (15, 95) est précontrainte en direction de sa première position axiale (88).

12. Appareil d'injection selon la revendication 11,
**caractérisé en ce que** la partie de blocage (15, 95) présente au moins un bras de ressort (96), qui précontraint la partie de blocage (15, 95) en direction de sa première position axiale (88).

13. Appareil d'injection selon l'une quelconque des revendications 1 à 12,
**caractérisé en ce qu'**entre la gaine d'injection (17, 97) et l'organe de dosage (18) agit un ressort (23, 93), qui précontraint l'organe de dosage (18) dans la deuxième direction de rotation.

14. Appareil d'injection selon la revendication 13,
**caractérisé en ce que** le ressort (93) est fixé avec une extrémité (84) à la gaine d'injection (97) et avec l'autre extrémité (85) à l'élément d'entraînement (14).

15. Appareil d'injection selon la revendication 13,
**caractérisé en ce que** le ressort (23) est fixé avec une extrémité (84) à la partie de blocage (15) et avec l'autre extrémité (85) à l'élément d'entraînement (14).
